# EUROPEAN PATENT SPECIFICATION

(11) **EP 0 902 016 B1**
(45) Date of publication and mention of the grant of the patent: **02.05.2002**
(21) Application number: 98115155.8
(22) Date of filing: 12.08.1998
(51) Int. Cl.: C07D 211/90, C07C 69/24, C07C 69/618, C07D 487/18

(54) **A process and intermediate compounds for the preparation of amlodipine benzene sulphonate**
Verfahren und Zwischenprodukte zur Herstellung von Amlodipin-Benzolsulfonat
Procédé et produits intermediaires pour la préparation du benzénesulfonate d'amlodipine

(30) Priority: 12.08.1997 HU 9701379
(43) Date of publication of application: 17.03.1999
(73) Proprietor: EGIS GYOGYSZERGYAR RT., 1106 Budapest (HU)
(72) Inventor: Bozsing, Dániel, Dr., 1141 Budapest (HU); Koványi, Györgyi, Lax, Dr., 1141 Budapest (HU); Simig, Gyula, Dr., 1126 Budapest (HU); Krasznai, György, 1172 Budapest (HU); Blasko, Gábor, Dr., 1149 Budapest (HU); Tömpe, Péter, Dr., 1116 Budapest (HU); Nagy, Kálmán, Dr., 1025 Budapest (HU); Vereczkey, Györgyi Donáth, 1034 Budapest (HU); Német, Gábor, 1149 Budapest (HU); Németh, Norbert, 1119 Budapest (HU)
(74) Representative: Beszédes, Stephan G., Dr.

(56) References cited:
- EP-A- 0 089 167
- EP-A- 0 599 220
- WO-A-91/15484
- KAUFFMAN J.M. ET AL.: "Syntheses and Photophysical Properties of Some 5(2)-Aryl-2(5)-(4-pyridyl)oxazoles and Related Oxadiazoles and Furans" J.HETEROCYCL.CHEM., vol. 29, no. 5, 1992, pages 1245-1273, XP002084239

## Description

The present invention is concerned with a new process and new intermediates for preparing 3-[ethyl]-5-[methyl]-2-[2'-(aminoethoxy)-methyl)-4-[2"-(chloro)-phenyl]-6-[methyl]-1,4-di- [hydro]-pyridine-3,5-di-[carboxylate] benzenesulfonic acid salt. This compound is a known pharmaceutically active ingredient having the INN amlodipine besylate.

Amlodipine besylate is a calcium antagonist of the dihydropyridine-dicarboxylate type and possesses valuable strong long-lasting blood pressure decreasing and antianginal properties.

According to the known processes the dihydropyridine structure of amlodipine is built up by means of the Hantzsch synthesis suggested by the Prior Art for reactions of this type. The main feature of the process disclosed in EP-A-89,167 and HU 186,868 is that the primary amino group is formed in the last step of the synthesis either by removing the protecting group from a protected amino group or by reducing the corresponding azide. The proteced amino group or the azido group is introduced into the molecule by incorporating into the acetoacetate component of the Hantzsch synthesis.

According to the first process mentioned above in the Hantzsch synthesis a 4-[2'-(amino)-ethoxy]-acetoacetic ester bearing a protected amino group, 2-[chloro]-benzaldehyde and an amino crotonic acid ester were reacted, or in a variant of said process a 4-[2'-(amino)-ethoxy]-acetoacetic ester was condensed first with a 2-[chloro]-benzaldehyde and the "ylidine" derivative thus obtained was reacted with an amino crotonic acid ester. Amlodipine was prepared by removing the protecting group from the dihydropyridine derivative containing a protected primary amino group obtained in the Hantzsch synthesis.

According to the other process referred to Hantzsch synthesis it was performed by reacting a 4-[2'-(azido)-ethoxy]-acetoacetic acid ester, 2-chloro-benzaldehyde and amino crotonic acid ester. The primary amino group of amlodipine was formed by reducing the azido group.

In the EP-A-89,167 cited above pharmaceutically acceptable acid addition salts of amlodipine were disclosed. Said salts were prepared from amlodipine by salt formation. Among the salts the maleate has been described as the most advantageous one.

The disadvantage of the above processes is the relatively low yield of the individual reaction steps (the yield of the Hantzsch synthesis has not been even disclosed). It is known furtheron that the azide is explosive (see citation C.A. 105, 11321t relating to the relevant azido compound).

In DE 3,710,457 the benzenesulfonic acid salt of amlodipine and a process for preparing it have been disclosed. According to this patent said salt exhibits numerous advantages over other known salts of amlodipine, particularly in the incorporation of the salt into pharmaceutical compositions. Amlodipine besylate was prepared by reacting amlodipine base with a solution of benzenesulfonic acid or the ammonium salt thereof formed with an inert solvent and isolating the amlodipine besylate thus obtained from the reaction mixture.

In EP-A-599,220 a further process for preparing amlodipine besylate has been disclosed. An amiodipine derivative containing a trityl protective group on the primary amino group was prepared by using the conventional Hantzsch synthesis. The protecting trityl group was removed by hydrolysis carried out in the presence of benzenesulfonic acid. Thus amlodipine besylate is obtained without isolating the amlodipine base. The significant drawback of this process resides in the low yield. It is a further important disadvantage that pure end-product can only be obtained with the aid of extremely complicated measures. The overall yield is only 7%, related to 2-[chloro]-benzaldehyde.

The methods of EP-A-89,167 and EP-A-599,220 follow the strategy of reacting an ethyl 4-[2'-(substituted-ethoxy)]-acetoacetate with 2-(chloro)-benzaldehyde and a methyl 3-(amino)-crotonate in order to obtain a 3-ethyl 5-methyl-2-[2'-(substituted-ethoxy)-methyl]-4-[2"-chlorophenyl]-6-methyl-1,4-dihydropyridine-3,5-dicarboxylate intermediate. The substituent in "the substituted-ethoxy" residue of the obtained intermediate is converted to an amino moiety in order to obtain the end compound in a form of the corresponding salt.

In WO 91 15484 A there has been described a method for preparing dihydropyridines having an amino moiety on a 2-ethoxymethyl substituent which has been introduced by an amine of formula HNR6aR6b.

KAUFMAN J.M. ET AL.: 'Syntheses and Photophysical Properties of Some 5(2)-Aryl-2 (5) - (4-pyridyl)oxazoles and Related Oxadiazoles and Furans' J.HETEROCYCL.CHEM., vol. 29, no. 5, 1992, pages 1245-1273, XP002084239 discloses the use of hexamethylene tetramine for forming an amino moiety in the form of an acid addition salt (= Delépine reaction in the preparation of aryl-pyridyl-substituted oxazoles.

WO 91 15484 A discloses intermediates which bear a 2-ethoxymethyl substituent in the 2-position of the dihydropyridine ring. The general definition of the intermediates of this publication encompasses 3-[ethyl]-5-[methyl] -2-[2'-(chloro)-ethoxy-methyl]-4-[2''-(chloro)-phenyl]-6-[methyl]-1,4-di-[hydro]-pyridine-3,5-di-[carboxylate] and 3-[ethyl]-5-[methyl]-2-[2'-(iodo)-ethoxy-methyl-4-[2''-(chloro)-phenyl]-6-[methyl]-1,4-di-[hydro]-pyridine-3,5-di-[carboxylate]. However, these have not been disclosed specifically.

The problem underlying to the invention is to provide for a new process and new intermediates for preparing 3-[ethyl]-5-[methyl]-2-[2'-(aminoethoxy)-methyl]-4-[2''-(chloro)-phenyl]-6-[methyl]-1,4-di-[hydro]-pyridine-3,5-di-[carboxylate] benzenesulfonic acid salt {amlodipine besylate} by which by overcoming the above disadvantages of the known processes this compound can be obtained with high yields and in a simple way and with elimination of the isolation of amlodipine base.

Surprisingly this has been achieved by the present invention.

A subject matter of the invention is a process for preparing 3-[ethyl]-5-[methyl]-2-[2'-(aminoethoxy)-methyl]-4-[2''-(chloro)-phenyl]-6-[methyl]-1,4-di-[hydro]-pyridine-3,5-di-[carboxylate] benzenesulfonic acid salt of Formula 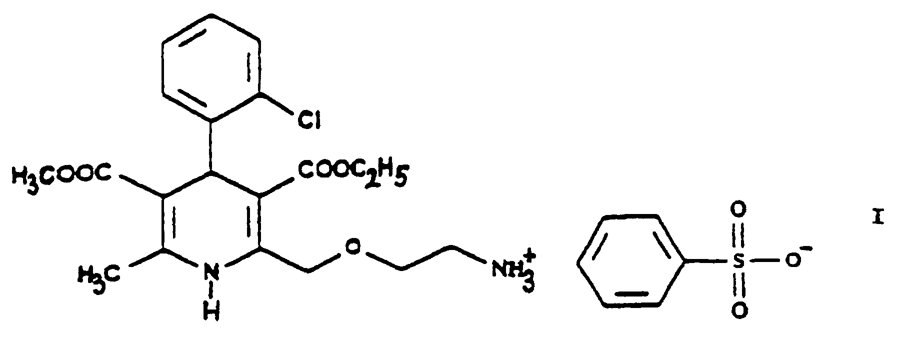 including its optically active isomers, which is characterized by
a₁) reacting 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula 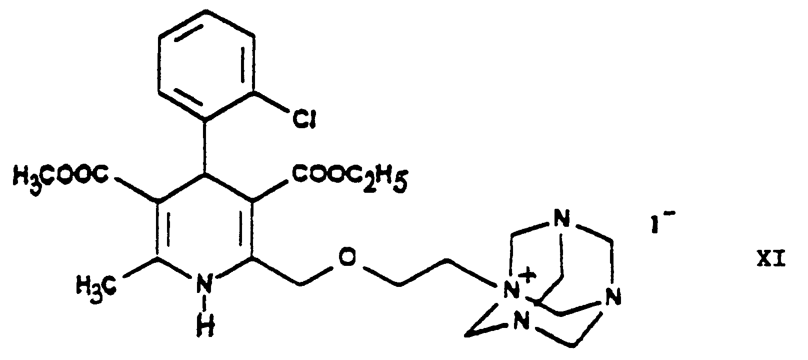 with benzenesulfonic acid of Formula 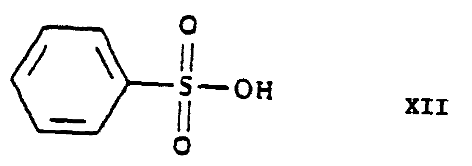 in a mixture of water and 1 or more organic solvent(s);
   or
a₂) reacting 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula 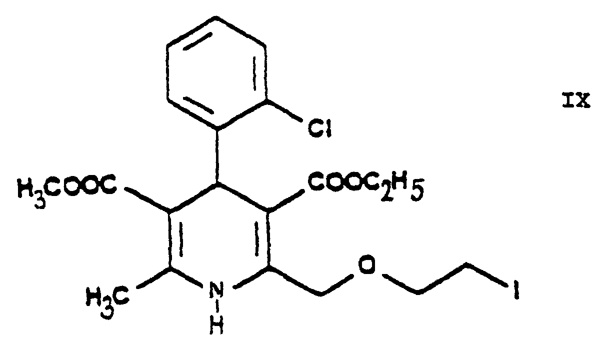 with hexamethylene tetramine of Formula 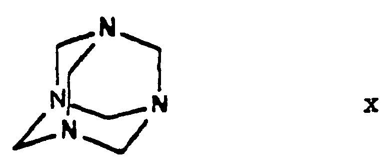 and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
   or
a₃) exchanging in 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula 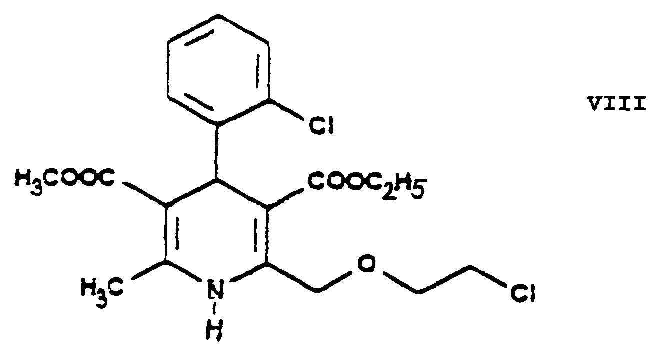 the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
   or
a₄) reacting ethyl-4-[2'-(chloro)-ethoxy]-2-[2''-- (chloro)-benzylidene]-acetoacetate of Formula 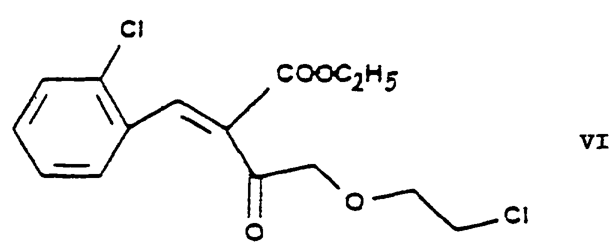 with methyl-3-(amino)-crotonate of Formula 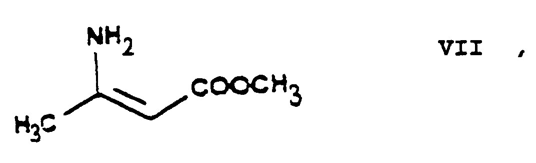 exchanging in the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
   or
a₅) reacting ethyl-4-[2'-(chloro)-ethoxy]-acetoacetate of Formula 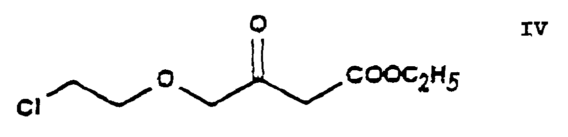 with 2-(chloro)-benzaldehyde of Formula 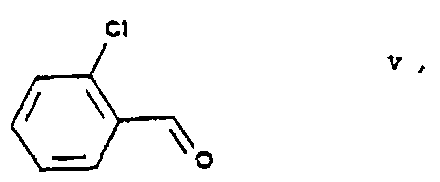 reacting the ethyl-4-[2'-(chloro)-ethoxy]-2-[2''-(chloro)-benzylidene]-acetoacetate of Formula VI thus obtained with methyl-3-(amino)-crotonate of Formula VII, exchanging in the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula X thus obtained with hexamethylene tetramine of Formula X and reacting the 3-[ethyl]-5-[methyl]-2-[2'-(yl)-ethoxy-methyl]-4-[2''-(chloro)-phenyl]--6-[methyl]-1,4-di-[hydro]-pyridine-3,5-di--[carboxylate]-hexaminium iodide of Formula XI thus obtained with benzenesulfonic acid of Formula XII;
   or
a₆) reacting ethyl-4-(bromo)-acetoacetate of Formula 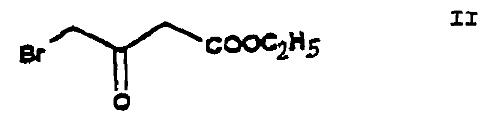 with ethylene chlorohydrine of Formula 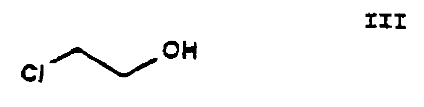 reacting the ethyl-4-[2'-(chloro)-ethoxy]-acetoacetate of Formula IV thus obtained with 2-(chloro)-benzaldehyde of Formula V, reacting the ethyl-4-[2'-(chloro)-ethoxy]-2-[2''-(chloro)-benzylidene]-acetoacetate of Formula VI thus obtained with methyl-3-(amino)-crotonate of Formula VII, exchanging in the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
   or
a₇) exchanging in the ethyl-4-[2'-(chloro)-ethoxy]-acetoacetate of Formula IV the chlorine for iodine, reacting the ethyl-4-[2'-(iodo)-ethoxy]-acetoacetate of Formula 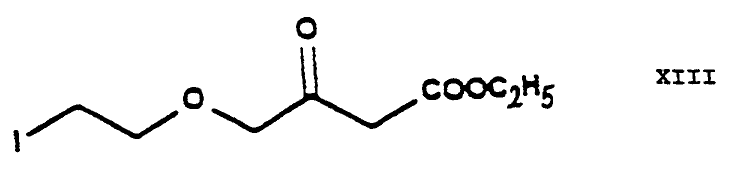 thus obtained with 2-(chloro)-benzaldehyde of Formula V, reacting the ethyl-4-[2'-(iodo)-ethoxy]-2-[2''-(chloro)-benzylidene]-acetoacetate of Formula 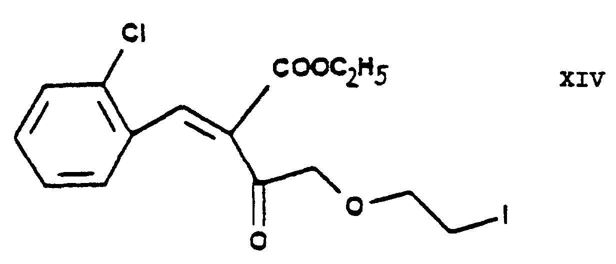 thus obtained with methyl-3-(amino)-crotonate of Formula VII, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl] - -4-[2''-(chloro)-phenyl]-6-[methyl]-1,4-di-[hydro]--pyridine-3,5-di-[carboxylate]-hexaminium iodide of Formula XI thus obtained with benzenesulfonic acid of Formula XII,
in variants a₅) and a₆) alternatively reacting the ethyl-4-[2'-(chloro)-ethoxy]-acetoacetate of Formula IV with the 2-(chloro)-benzaldehyde of Formula V and the methyl-3-(amino)-crotonate of Formula VII to the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII without isolating the ethyl-4-[2'-(chloro)-ethoxy]-2-[2''-(chloro)-benzylidene]-acetoacetate of Formula VI, optionally separating optically active isomers in a manner known per se from the 3-[ethyl]-5-[methyl]-2-[2'-(aminoethoxy)-methyl]-4-[2''-(chloro)-phenyl]-6-[methyl]-1,4-di-[hydro]-pyridine-3,5-di-[carboxylate] benzenesulfonic acid salt of Formula I, the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII, the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX or the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained and further reacting the optically active isomers of the last three ones thus obtained.

The difference between the method according to the invention and the methods of EP-A-89,167 and EP-A-599,220 arises from the fact that in the present method the said amino moiety is introduced into the 2-ethoxymethyl substituent via the hexaminium iodide intermediate of formula XI, whereas in the methods of the best-named references the said amino moiety is already present in protected form or in the form of N₃ in the acetoacetate starting material.

The present method differs from the method of WO 91 15484 A in that in the former hexamethylene tetramine has been used for introducing the amino moiety into the 2-ethoxymethyl substituent, whereas in the latter an amine residue HNR6aR6b has been used.

The intermediates 3-[ethyl]-5-[methyl]-2-[2'-(chloro)-ethoxy-methyl]-4-[2''-(chloro) -phenyl]-6-[methyl]-1,4-di-[hydro]-pyridine-3,5-di-[carboxylate]and 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] represent a selection from the intermediates of WO 91 15484 A made in several directions.

The essential feature of the present invention is that in the Hantzsch synthesis it is the benzylidene derivative ethyl-4-[2'-(chloro)-ethoxy]-2-[2"-(chloro)-benzylidene]-acetoacetate of Formula VI prepared from ethyl-4-[2'-(chloro)-ethoxy]-acetoacetate of Formula IV and 2-(chloro)-benzaldehyde of Formula V which is reacted with the amino crotonic acid ester methyl-3-(amino)-crotonate of Formula VII whereby the new 2-(chloroethoxy)-dihydropyridine derivative 3-[ethyl]-5-[methyl]-2-[2'-(chloro)-ethoxy-methyl]-4-[2"- (chloro)-phenyl]-6-[methyl]-1,4-di-[hydro]-pyridine-3,5-di-[carboxylate] of Formula VIII and after chlorine→iodine exchange the new 2-(iodoethoxy)-dihydropyridine derivative 3-[ethyl]-5-[methyl]-2-[2'-(iodo)-ethoxy-methyl]-4-[2"-(chloro)- phenyl]-6-[methyl]-1,4-di-[hydro]-pyridine-3,5-di-[carboxylate] of Formula IX are obtained with good yields.

The compounds 3-[ethyl]-5-[methyl]-2-[2'-(chloro)-ethoxy-methyl]-4-[2"-(chloro)-phenyl]-6-[methyl]-1,4-di-[hydro]-pyridine-3,5-di-[carboxylate] and 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formulae VIII and IX, respectively, are new, not described in the Prior Art. In this way 3-[ethyl]-5-[methyl]-2-[2'-(aminoethoxy)-methyl]-4-[2"-(chloro)-phenyl]-6-[methyl]-1,4-di-[hydro]-pyridine-3,5-di-[carboxylate] benzenesulfonic acid salt {amlodipine besylate} is prepared by a new and very advantageous method by ammonolysis of the halogeno derivative 3-[ethyl]-5-[methyl]-2-[2'-(iodo)-ethoxy-methyl]-4-[2"-(chloro)- phenyl]-6-[methyl]-1,4-di-[hydro]-pyridine-3,5-di-[carboxylate] of Formula IX by means of the so-called Delepine reaction. The halogen iodine is ultimately replaced by the amino group by using hexamethylene tetramine as ammonia source and converted into an iodide anion. The urotropine salt 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI formed is decomposed by using benzenesulfonic acid whereby 3-[ethyl]-5-[methyl]-2-[2'-(aminoethoxy)-methyl]-4-[2"-(chloro)-phenyl]-6-[methyl]-1,4-di-[hydro]-pyridine-3,5-di-[carboxylate] benzenesulfonic acid salt {amlodipine besylate} of Formula I is directly isolated from the reaction mixture. Also the latter process is new, not disclosed in the Prior Art.

According to the process of the present invention - contrary to methods described in the Prior Art - the primary amino group is not built up by introducing it into the molecule as a part of the acetoacetic acid ester component in protected form and subsequently removing the protecting group of the amino group but rather by a halogen→amine exchange reaction carried out after the Hantzsch reaction performed by using the corresponding halogeno compound. According to a characteristic essential element of the process of the present invention the latter reaction is carried out in a manner not disclosed in the Prior Art whereby in the reaction not amlodipine base but directly the desired besylate salt 3-[ethyl]-5-[methyl]-2-[2'-(aminoethoxy)-methyl]-4-[2"-(chloro)-phenyl]-6-[methyl]-1,4-di-[hydro]-pyridine-3,5-di-[carboxylate] benzenesulfonic acid salt is formed. The process is shown on the following reaction scheme.

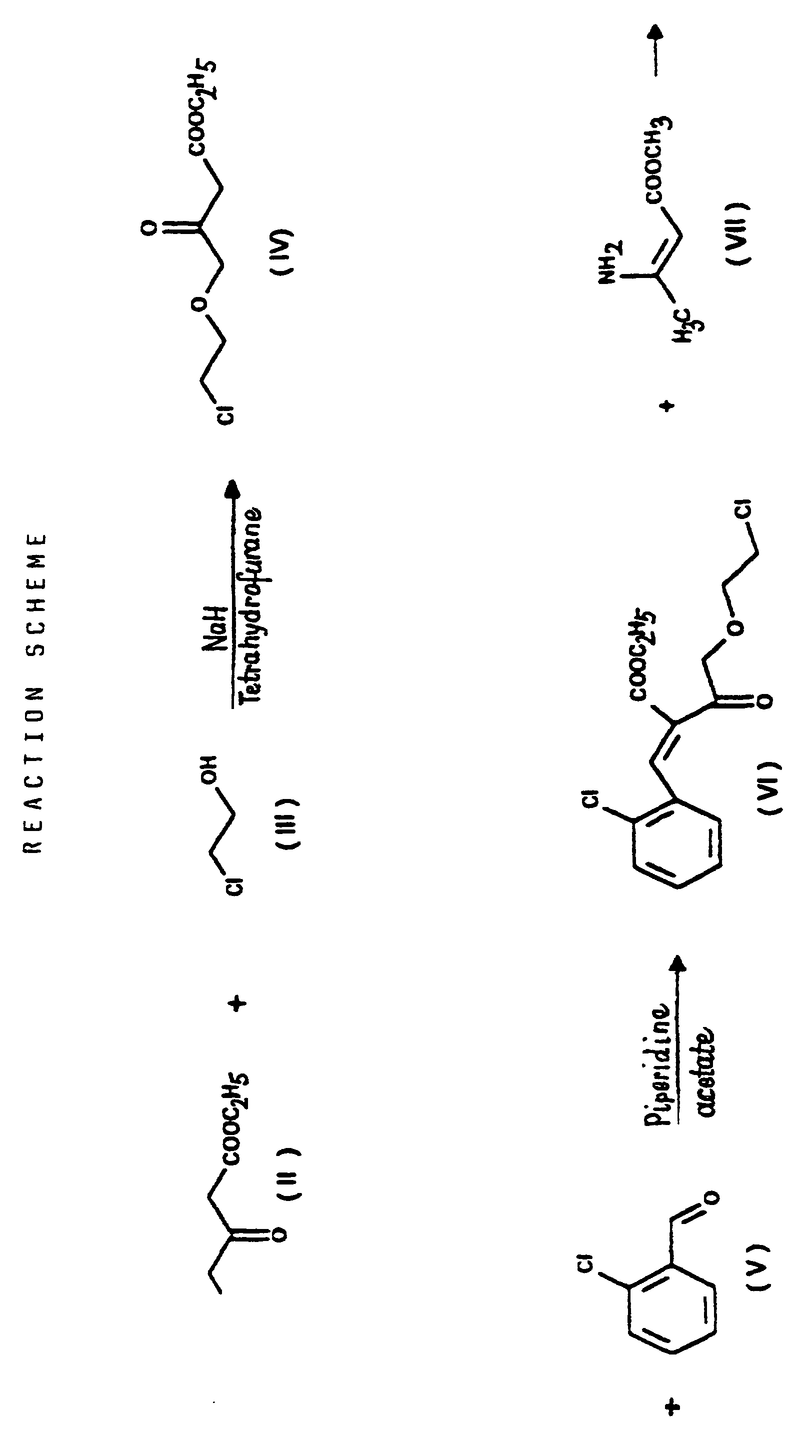

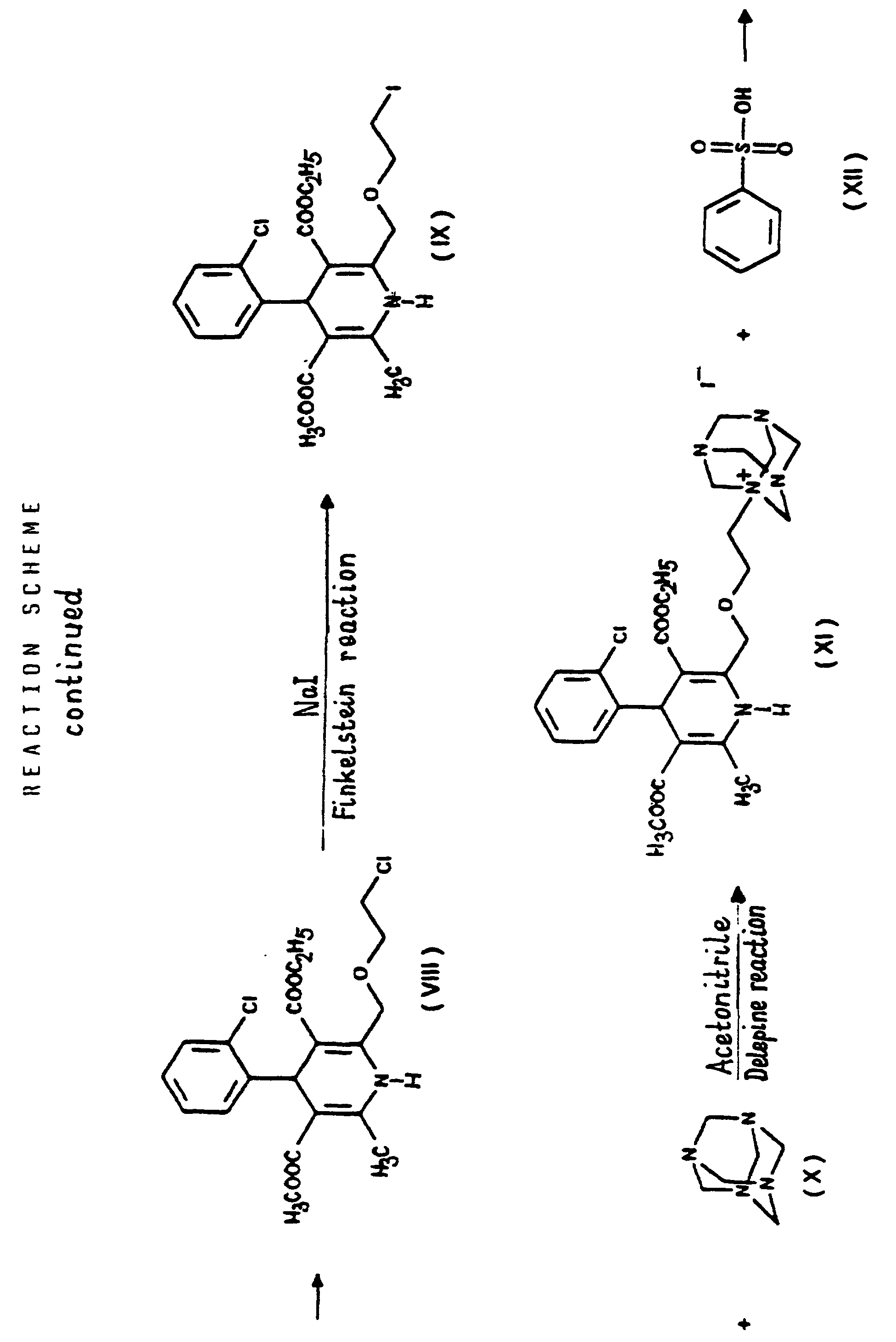

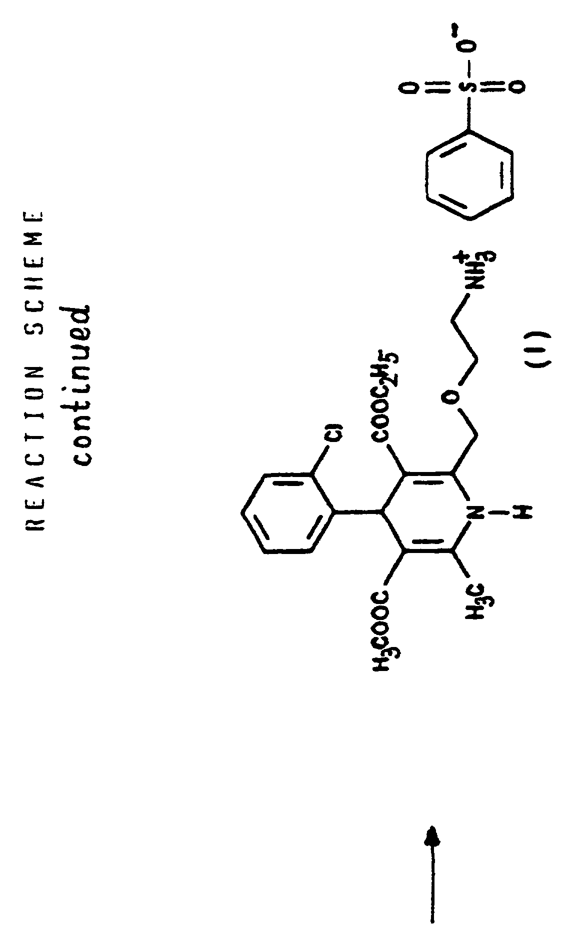

The reaction of the process of the present invention in which ethylene chlorohydrine [2-(chloro)-ethanol] of Formula III is O-alkylated with ethyl-4-(bromo)-acetoacetate of Formula II results in the new compound ethyl-4-[2'-(chloro)-ethoxy]-acetoacetate of Formula IV. The reaction can be carried out in a known manner. The reaction can be performed in an inert solvent, preferably 1 or more aliphatic and/or alicyclic ether(s), particularly tetrahydrofurane, at a temperature of about -10°C or below this temperature. Advantageously the hydrogen bromide formed is bound with a basic substance, preferably with sodium hydride, which can be used in the form of an oily suspension or can be made previously free of paraffine. The reaction mixture may be worked up in a known manner by decomposing with an acid, neutralization and extraction. The pure product may be obtained by fractionated distillation in vacuo.

As reaction product of the aldol condensation of the chloroethoxy-acetoacetic acid ester ethyl-4-[2'-(chloro)-ethoxy]-acetoacetate of Formula IV with 2-(chloro)-benzaldehyde of Formula V the "ylidene" derivative ethyl-4-[2'-(chloro)-ethoxy]-2-[2"-(chloro)-benzylidene]- acetoacetate of Formula VI is obtained. Preferably this reaction is carried out in the presence of piperidine acetate as catalyst, advantageously in a catalytic amount of the latter, whereby the ethyl-4-[2'-(chloro)-ethoxy]-2-[2"-(chloro)-benzylidene]-acetoacetate of Formula VI is obtained in very high yield. The piperidine acetate catalyst is preferably used in a 0.01 to 0.1 molar amount, related to 1 mole of the ethyl-4-[2'-(chloro)-ethoxy]-acetoacetate of Formula IV. As reaction medium advantageously 1 or more protic solvent(s) may be used, preferably 1 or more alkanol(s), particularly isopropanol. Advantageously as reaction temperatures from 10°C to 60°C are applied, one may preferably work at room temperature. The reaction time can be varied from 5 to 15 hours and preferably 10 hours are chosen. The reaction mixture may be worked up by evaporating the solvent and washing with water. The crude product ethyl-4-[2'-(chloro)-ethoxy]-2-[2"-(chloro)-benzylidene]--acetoacetate of Formula VI thus obtained is generally suitable for further chemical transformation.

One may also proceed by subjecting the ethyl-4-[2'-(chloro)-ethoxy]-2-[2"-(chloro)-benzylidene]-acetoacetate of Formula VI to further chemical reaction directly, without isolation, in the solvent used.

In the preparation of the 3-[ethyl]-5-[methyl]-2-[2'-(chloro)-ethoxy-methyl]-4-[2"-(chloro)-phenyl]-6-[methyl]-1,4-di-[hydro]-pyridine-3,5-di-[carboxylate] of Formula VIII by Hantzsch synthesis the ethyl-4-[2'-(chloro)-ethoxy]-2-[2"-(chloro)-benzylidene]-acetoacetate of Formula VI is reacted with methyl-3-(amino)-crotonate of Formula VII one may advantageously proceed by heating to boiling the said reactants. Furthermore it is advantageous to carry out this reaction in an organic solvent. Preferably the reaction of the ethyl-4-[2'-(chloro)ethoxy]-2-[2"-(chloro)-benzylidene]-acetoacetate of Formula VI with the methyl-3-(amino)-crotonate of Formula VII is carried out in 1 or more C₁₋₄-alkanol(s), particularly methanol, ethanol and/or isopropanol, and/or polar aprotic organic solvent(s), particularly acetonitrile.

One may also proceed by reacting 2-(chloro)-benzaldehyde of Formula V, the keto ester ethyl-4-[2'-(chloro)-ethoxy]-acetoacetate of Formula IV and the amino crotonate methyl-3-(amino)-crotonate of Formula VII without isolating the benzylidene derivative ethyl-4-[2'-(chloro)-ethoxy]-2-[2"-(chloro)-benzylidene]-acetoacetate of Formula VI until the reaction becomes complete. As reaction time advantageously from 15 to 20 hours or shorter are chosen. The reaction mixture can be worked up in a known manner, e.g. by cooling and filtration.

The reaction of exchanging in the 3-[ethyl]-5-[methyl]-2-[2'-(chloro)-ethoxy-methyl]-4-[2"-(chloro)-phenyl]-6-[methyl]-1,4-di-[hydro]-pyridine-3,5-di-[carboxylate] of Formula VIII the chlorine for iodine to result in the 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formula IX is the "Finkelstein reaction" known from the Prior Art per se. This reaction is preferably carried out by reacting the 3-[ethyl]-5-[methyl]-2-[2'-(chloro)-ethoxy-methyl]-4-[2"-(chloro)-phenyl]-6-[methyl]-1,4-di-[hydro]-pyridine-3,5-di-[carboxylate] of Formula VIII with 1 or more alkali iodide(s), particularly sodium iodide. According to the teaching of the Prior Art this reaction is carried out preferably in acetone in view of the solubility conditions. The process of the present invention may be performed in acetone but the use of 1 ore more alkanol(s) having a high boiling point, particularly isopropanol, proved to be particularly advantageous. The reaction may be carried out under heating, preferably at the boiling point of the reaction mixture. The reaction time is chosen generally from 20 to 25 hours or shorter. The reaction mixture may be worked up in a known manner, e.g. by filtration after intensive cooling. The dihydropyridine derivative 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formula IX is obtained with good yield.

According to an alternative embodiment of the process of the present invention the chlorine-iodine exchange is carried out at the stage of the chloroethoxy acetoacetate ethyl-4-[2'-(chloro)-ethoxy]-acetoacetate of Formula IV. This reaction gives the iodo-acetoacetate ethyl-4-[2'-(iodo)-ethoxy]-acetoacetate of Formula XIII. The reaction is preferably carried out in acetone as medium at the boiling point of the reaction mixture. Furthermore preferably this chlorine-iodine exchange is carried out by reacting the ethyl-4-[2'-(chloro)-ethoxy]-acetoacetate of Formula IV with 1 or more alkali iodide(s), particularly sodium iodide. The product may be purified by fractionated distillation. The reaction of the ethyl-4-[2'-(iodo)-ethoxy]-acetoacetate of Formula XIII thus obtained with the 2-(chloro)-benzaldehyde of Formula V to yield the benzylidene derivative ethyl-4-[2'-(iodo)-ethoxy]-2-[2"-(chloro)-benzylidene]-acetoacetate of Formula XIV is advantageously carried out as described in connection with the reaction of the ethyl-4-[2'-(chloro)-ethoxy]-acetoacetate of Formula IV with the 2-(chloro)-benzaldehyde of Formula V. The reaction of the ethyl-4-[2'-(iodo)-ethoxy]-2-[2"-(chloro)-benzylidene]-acetoacetate of Formula XIV thus obtained with the methyl-3-(amino)-crotonate of Formula VII to yield the iodoethoxy-dihydropyridine derivative 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formula IX is advantageously carried out in a manner analogous to the Hantzsch reaction of the ethyl-4-[2'-(chloro)-ethoxy]-2-[2"-(chloro)-benzylidene]-acetoacetate of Formula VI with the methyl-3-(amino)-crotonate of Formula VII.

The reaction of the iodo derivative 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formula IX - this being more reactive than the chloroethoxy-dihydropyridine 3-[ethyl]-5-[methyl]-2-[2'- (chloro)-ethoxy-methyl]-4-[2"-(chloro)-phenyl]-6-[methyl]-1,4-di-[hydro]-pyridine-3,5-di-[carboxylate] of Formula VIII with hexamethylene tetramine (urotropine) of Formula X is the formation of a quaternary salt, namely 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI. According to the teaching of the Prior Art salts of such type are generally prepared in apolar-aprotic solvents. However, it has been found, that the said reaction of the process according to the invention may be carried out more advantageously by using 1 or more C₁₋₅-alkanol(s), e.g. methanol, ethanol and/or isopropanol, and/or acetonitrile as reaction medium. One may work particularly preferably in acetonitrile as medium. The reactants may be used in equimolar amount, but hexamethylene tetramine (urotropine) of Formula X may also be used preferably in an excess of 10 to 15%. The reaction may be carried out advantageously at temperatures from room temperature to the boiling point of the solvent, preferably at 40 to 55°C, particularly 45 to 50°C. One may proceed by adding the reaction components simultaneously to the solvent or adding the iodo compound 3-[ethyl]-5-[methyl]-2-[2'-(iodo)-ethoxy-methyl]-4-[2"-(chloro)-phenyl]-6-[methyl]-1,4-di-[hydro]-pyridine-3,5-di-[carboxylate] of Formula IX to the solution of hexamethylene tetramine, preferably in small portions. As reaction time advantageously 20 to 50 hours, preferably 30 to 40 hours, are chosen. The dihydropyridine-urotropine quaternary salt 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI precipitates as a solid and can be simply filtered off at room temperature. The crude product is of a purity suitable for the preparation of the end-product and no further purification is required.

The reaction of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI with benzenesulfonic acid of Formula XII to yield 3-[ethyl]-5-[methyl]-2-[2'-(aminoethoxy)-methyl]-4-[2"-(chloro)-phenyl]-6-[methyl]-1,4-di-[hydro]-pyridine-3,5-di-[carboxylate] benzenesulfonic acid salt {amlodipine besylate} is a hydrolysis.

As already indicated, the hydrolysis of the 3-[ethyl]-5-[methyl]-2- [2'-(yl)-ethoxy-methyl] -4- [2"- (chloro) -phenyl]-6-[methyl]-1,4-di- [hydro]-pyridine-3,5-di-[carboxylate]-hexaminimum iodide of Formula XI with benzenesulfonic acid of Formula XII is carried out in a mixture of water and 1 or more organic solvent(s). According to one alternative 1 or more water miscible organic solvent(s) is/are used as [an] organic solvent(s) for the hydrolysis of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminimum iodide of Formula XI with benzenesulfonic acid of Formula XII. Preferably 1 or more straight or branched chained C₁₋₃-alkanol(s), e.g. methanol, ethanol and/or isopropanol, is/are used as [a] water miscible solvent(s) for the hydrolysis of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminimum iodide of Formula XI with benzenesulfonic acid of Formula XII.

According to another alternative 1 or more organic solvent(s) partially miscible or immiscible with water is/are used as [an] organic solvent(s) for the hydrolysis of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminimum iodide of Formula XI with benzenesulfonic acid of Formula XII. In this alternative preferably 1 or more C₄₋₈-alkanol(s), particularly n-butanol, and/or ethyl acetate is/are used as [an] organic solvent(s) for the hydrolysis of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminimum iodide of Formula XI with benzenesulfonic acid of Formula XII. The reaction may be carried out advantageously at temperatures from room temperature to the boiling point of the solvent; one may preferably work at the boiling point of the reaction mixture. Preferably benzenesulfonic acid of Formula XII is used in at least a 4 molar amount, related to 1 mole of the 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI. For practical reasons it is preferred to use not more than 10 molar equivalents of benzenesulfonic acid of Formula XII. According to a particularly preferred embodiment of the process the reaction is carried out by using about 5 molar equivalents of benzenesulfonic acid of Formula XII. The reaction mixture may be worked up in a manner known per se.

The starting materials ethylene chlorohydrine of Formula III, 2-(chloro)-benzaldehyde of Formula V, hexamethylene tetramine of Formula X and benzenesulfonic acid of Formula XII are commercially available. The ethyl-4-(bromo)-acetoacetate of Formula II is a known compound and can be prepared e.g. according to US 3,786,082 and EP 102,893. The methyl-3-(amino)-crotonate of Formula VII has been known as well (HU 202,474).

The intermediates ethyl-4-[2'-(chloro)-ethoxy]-acetoacetate of Formula IV, ethyl-4-[2'-(chloro)-ethoxy]-2-[2"-(chloro)-benzylidene]-acetoacetate of Formula VI, 3-[ethyl]-5-[methyl]-2-[2'-(chloro)-ethoxy)-methyl]-4-[2"-(chloro)-phenyl]-6-[methyl]-1,4-di-[hydro]-pyridine-3,5-di-[carboxylate] of Formula VIII, 3-[ethyl]-5-[methyl]-2-[2'-(iodo)-ethoxy)-methyl]-4-[2"-(chloro)-phenyl]-6-[methyl]-1,4-di-[hydro]-pyridine-3,5-di-[carboxylate] of Formula IX, 3-[ethyl]-5-[methyl]-2-[2'-(yl)-ethoxy-methyl]-4-[2"-(chloro)-phenyl]-6-[methyl]-1,4-di-[hydro]-pyridine-3, 5-di-[carboxylate]-hexaminium iodide of Formula XI, ethyl-4-[2'-(iodo)-ethoxy]-acetoacetate of Formula XIII and ethyl-4-[2'-(iodo)-ethoxy]-2-[2"-(chloro)-benzylidene]-acetoacetate of Formula XIV are new compounds, not disclosed in the Prior Art.

Hence a further subject matter of the present invention are the new compounds ethyl-4-[2'-(chloro)-ethoxy]-acetoacetate of Formula IV, ethyl-4-[2'-(chloro)-ethoxy]-2-[2"-(chloro)-benzylidene]-acetoacetate of Formula VI, 3-[ethyl]-5-[methyl]-2-[2'-(chloro)-ethoxy)-methyl]-4-[2"-(chloro)-phenyl]-6-[methyl]-1,4-di-[hydro]-pyridine-3,5-di-[carboxylate] of Formula VIII, 3- [ethyl] -5-[methyl]-2-[2'-(iodo)-ethoxy)-methyl]-4-[2"-(chloro)-phenyl]-6-[methyl]-1,4-di-[hydro]-pyridine-3,5-di-[carboxylate] of Formula IX, 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI, ethyl-4-[2'-(iodo)-ethoxy]-acetoacetate of Formula XIII and ethyl-4-[2'-(iodo)-ethoxy]-2- [2"-(chloro)-benzylidene]- acetoacetate of Formula XIV which are valuable intermediates via which the end product 3-[ethyl]-5-[methyl]-2-[2'-(aminoethoxy)-methyl]-4-[2"-(chloro)-phenyl]-6-[methyl]-1,4-di-[hydro]-pyridine-3,5-di-[carboxylate] benzenesulfonic acid salt {amlodipine besylate} can be prepared by a process having originality in a superior way.

These intermediates can be prepared by stopping the respective variants of the process according to the invention at the suitable stage.

Compounds of the dihydropyridine dicarboxylate structure are mixed esters and contain an asymmetrical center. Such compounds may occur in the form of a pair of enantiomers, which can be separated by methods well-known from the Prior Art. The present invention also encompasses the preparation of the individual isomers (dextro and laevo rotatory isomers) and mixtures thereof (including racemic mixtures), namely of 3-[ethyl]-5-[methyl]-2-[2'-(aminoethoxy)-methyl]-4-[2"-(chloro)-phenyl]-6-methyl]-1,4-di-[hydro]-pyridine-3,5-di-[carboxylate] benzenesulfonic acid salt {amlodipine besylate} of Formula I, 3-[ethyl]-5-[methyl]-2-[2'-(chloro)-ethoxy)-methyl]-4-[2"-(chloro)-phenyl]-6-[methyl]-1,4-di-[hydro]-pyridine-3,5-di-[carboxylate] of Formula VIII, 3-[ethyl]-5-[methyl]-2-[2'-(iodo)-ethoxy)-methyl]-4-[2"-chloro)-phenyl]-6-[methyl]-1,4-di-[hydro]-pyridine-3,5-di-[carboxylate] of Formula IX and 3-[ethyl]-5-[methyl]-2-[2'-(yl-ethoxy)-methyl]-4-[2"-(chloro)-phenyl]-6-[methyl]-1,4-di-[hydro]-pyridine-3,5-di-[carboxylate]-hexaminium iodide of Formula XI.

Also the hydrolysis of the quaternary salt 3-[ethyl]-5-[methyl]-2-[2'-(yl)-ethoxy-methyl]-4-[2"-(chloro)-phenyl]-6-[methyl]-1,4-di-[hydro]-pyridine-3,5-di-[carboxylate]-hexaminium iodide of Formula XI with benzenesulfonic acid is a new process having originality.

The main advantage of the present invention is that the yields of the individual steps are high. Thus also the yield of the Hantzsch cyclisation step used in the process according to the invention is higher than that of the known cyclisation reactions leading to amlodipine. A further advantage of the invention resides in the fact that there is no need to isolate the amlodipine base because the precipitated salt is directly formed in one step. The process is feasible on industrial scale too, no special equipment being required.

Further details of the present invention are to be found in the following Examples.

### Example 1

### Ethyl-4-[2'-(chloro)-ethoxy]-acetoacetate (IV)

10.38 g (0.25 mole) of a 57.8% by weight paraffine only suspension of sodium hydride are added to 110 ml of tetrahydrofurane. The mixture is cooled to a temperature of from -10°C to -20°C whereupon at this temperature 10.08 g (0.125 mole) of ethylene chlorohydrine (III) are added under nitrogen dropwise. The mixture is stirred for 20 minutes, whereupon at the same temperature a solution of 26.18 g (0.125 mole) of ethyl-4-(bromo)-acetoacetate (II) and 35 ml of tetrahydrofurane is added. The reaction mixture is stirred for 20 minutes, allowed to warm to room temperature, kept at this temperature for 6 hours, poured into 270 ml of 1 N hydrochloric acid under cooling and extracted with dichloro methane. The organic layer is dried and evaporated. The residual oil is made free of paraffine by treatment with a mixture of acetonitrile and benzene in a ratio by volume of 1 : 1. The product is distilled off in vacuo. Thus 17.47 g of the desired compound are obtained, yield 67%,
bp.: 110°C/2 Hg mm.

| Elementary analysis: for the Formula C₈H₁₃ClO₄ (208,41): | | | |
|---|---|---|---|
| calculated | C 46.05%, | H 6.28%, | Cl 16.99%; |
| found | C 46.45%, | H 6.11%, | Cl 16.52%. |

### Example 2

### Ethyl-4-[2'-(iodo)-ethoxy]-acetoacetate (XIII)

To a solution of 19 g (91 millimoles) of ethyl-4-[2'-(chloro)-ethoxy]-acetoacetate (IV) and 380 ml of acetone 134.4 g (910 millimoles) of sodium iodide are added. The reaction mixture is heated to boiling for 13 hours. The inorganic substance is filtered off and the filtrate is evaporated in vacuo. The residual oil is dissolved in dichloro methane, the solution is washed with water, dried and evaporated. The crude product is subjected to fractionated distillation in vacuo. Bp.: 170°C/0.1 Hg mm. Thus 18.3 g of the desired product are obtained, yield 67%.

| Elementary analysis: for the Formula C₈H₁₃IO₄ (300.091) : | | |
|---|---|---|
| calculated | C 32.02%, | H 4.37%; |
| found | C 31.86%, | H 4.36%. |

### Example 3

### Ethyl-4-[2'-(chloro)-ethoxy]-2-[2''-(chloro)-benzylidene]-acetoacetate (VI)

16.64 g (0,118 mole) of 2-(chloro)-benzaldehyde (V) and 24.7 g (0,118 mole) of ethyl-4-[2'-(chloro)-ethoxy]-acetoacetate (IV) are reacted in 365 ml of isopropanol in the presence of a piperidine acetate catalyst [10 g (11,8 millimoles) of piperidine + 0.7 g (11,8 millimoles) of acetic acid] at room temperature for 10 hours. The reaction mixture is evaporated, the residual oil is dissolved in dichloro methane, washed with water and dried. The organic phase is evaporated in vacuo. Thus 37.9 g of the desired product are obtained in the form of a yellow oil, yield 97%.

| Elementary analysis: for the Formula C₁₅H₁₆Cl₂O₄ (331.203) | | | |
|---|---|---|---|
| calculated | C 54.39%, | H 4.87%, | Cl 21.41%; |
| found | C 53.69%, | H 5.03%, | Cl 20.98%. |

### Example 4

### Ethyl-4-[2'-(iodo)-ethoxy]-2-[2''-(chloro)-benzylidene]-acetoacetate (XIV)

10 g (33 millimoles) of ethyl-4-[2'-(iodo)-ethoxy]-acetoacetate (XIII) and 4.64 g (33 millimoles) of 2-(chloro)-benzaldehyde (V) are reacted in 100 ml of isopropanol in the presence of a piperidine acetate catalyst [0.28 g (3.3 millimoles) of piperidine + 0.198 g (3.3 millimoles) of acetic acid] at room temperature for 10 hours. The reaction mixture is evaporated, the residual oil dissolved in dichloro methane, washed with water and dried. The organic phase is evaporated in vacuo. Thus 11.55 g of the desired product are obtained in the form of a reddish brown oil, yield 83%.

| Elementary analysis: for the Formula C₁₅H₁₆ClIO₄ (422.643) : | | | |
|---|---|---|---|
| calculated | C 42.63%, | H 3.82%, | Cl 8.39; |
| found | C 43.00%, | H 4.12%, | Cl 8.13%. |

### Example 5

### 3-[Ethyl]-5-[methyl]-2-[2'-(chloro)-ethoxy-methyl]-4-- [2''-(chloro)-phenyl]-6-[methyl]-1,4-di-[hydro]--pyridine-3,5-di-[carboxylate] (VIII)

A mixture of 36 g (0.1087 mole) of ethyl-4-[2'-(chloro)-ethoxyl-2-[2''-(chloro)-benzylidene]-acetoacetate (VI) and 12.5 g (0.1087 mole) of methyl-3-(amino)-crotonate (VII) in 335 ml of isopropanol is reacted at the boiling point of the reaction mixture for 20 hours. The reaction mixture is cooled to a temperature of from 0°C to -5°C and allowed to stand in a refrigerator overnight. Next morning the precipitate is filtered, washed successively with cold isopropanol and diisopropyl ether. The crude product may be recrystallized from diisopropyl ether or aqueous acetic acid, if necessary. Thus 21.88 g of the desired product are obtained, yield 47%, mp.: 152 to 154°C.

| Elementary analysis: for the Formula C₂₀H₂₃Cl₂NO₅ (428,32): | | | | |
|---|---|---|---|---|
| calculated | C 56.08%, | H 5.41%, | N 3.27%, | Cl 16.56%; |
| found | C 56.10%, | H 5.42%, | N 3.37%, | Cl 16.18%. |

### Example 6

### 3-[Ethyl]-5-[methyl]-2-[2"-(iodo)-ethoxy-methyl]-4-[2''-(chloro)-phenyl]-6-[methyl]-1,4-di-[hydro]-pyridine-3,5-di-[carboxylate] (IX)

### Method a)

A mixture of 16 g (37 millimoles) of 3-[ethyl]-5-[methyl]-2-[2'-(chloro)-ethoxy-methyl]-4-[2''-(chloro)-phenyl]-6-[methyl]-1,4-di-[hydro]-pyridine-3,5-di [carboxylate] (VIII), 55.46 g (370 millimoles) of sodium iodide and 183 ml of isopropanol is stirred under boiling for 20 hours. The reaction mixture is cooled to a temperature of from 0°C to -5°C and stored in a refrigerator overnight. Next morning the precipitate is filtered off and washed with cold isopropanol. The crude product is recrystallized from isopropanol. Thus 16.35 g of the desired compound are obtained, yield 85%, mp.: 152 to 154°C.

| Elementary analysis: for the Formula C₂₀H₂₃ClINO₅ (519,76): | | | | |
|---|---|---|---|---|
| calculated | C 46.22%, | H 4.46%, | N 2.69%, | Cl 6.82%; |
| found | C 45.92%, | H 4.45%, | N 2.73%, | Cl 6.77%. |

### Method b)

A mixture of 11 g (26 millimoles) of ethyl-4-[2'-(iodo)-ethoxy]-2-[2''-(chloro)-benzylidene]-acetoacetate (XIV), 2.99 g (26 millimoles) of methyl-3-(amino)-crotonate (VII) and 110 ml of isopropanol is heated to boiling for 8 hours. The reaction mixture is evaporated, the residue crystallized from cold isopropanol, filtered and washed with cold isopropanol. The crude product is recrystallized from isopropanol. Thus 2.97 g of the desired compound are obtained, yield 22%, mp.: 152 to 155°C.

### Example 7

### 3-[Ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide (XI)

1.77 g (12.7 millimoles) of hexamethylene tetramine (X) are added to 15 ml of acetonitrile. The mixture is stirred at room temperature for 10 minutes, warmed to 45 to 50°C, whereupon 6.0 g (11,5 millimoles) of 3-[ethyl]-5-[methyl]-2--[2'-(iodo)-ethoxy-methyl]-4-[2''-(chloro)-phenyl]-6-[methyl]-1,4-di-[hydro]-pyridine-3,5-di-[carboxylate] (IX) are added in small portions within about 2 hours. The reaction mixture is stirred at this temperature for 40 hours, allowed to cool to room temperature, filtered and washed successively with acetonitrile and dichloro methane. Thus 6.83 g of the desired product are obtained in the form of a white powder, yield 90%, mp.: 177 to 179°C.

| : Elementary analysis: for the Formula C₂₆H₃₅ClIN₅O₅(659.957) | | | |
|---|---|---|---|
| calculated | C 47.32%, | H 3.35%, | N 10.61%; |
| found | C 46.84%, | H 5.42%, | N 10.40%. |

### Example 8

### 3-[Ethyl]-5-[methyl]-2-[2'-(aminoethoxy)-methyl]-4-[2''-(chloro)-phenyl]-6-[methyl]-1,4-di-[hydro]-pyridine-di-3,5-carboxylate] benzenesulfonic acid salt {amlodipine besylate} (I)

### Method a)

A mixture of 3.3 g (5 millimoles) of 3-[ethyl]-5-[methyl]-2-[2'-(yl)-ethoxy-methyl]-4-[2''-(chloro)-phenyl]--6-[methyl]-1,4-di-[hydro]-pyridine-3,5-di-[carboxylate]-hexaminium iodide (XI), 3.95 g (25 millimoles) of benzenesulfonic acid (XII), 350 ml of n-butanol and 350 ml of water is heated to boiling under vigorous stirring for 45 minutes. The reaction mixture is allowed to cool to room temperature and the layers are separated. The organic layer is washed with water, dried and evaporated.

The residual oil is crystallized by storing in cold ethyl acetate in a refrigerator overnight. Next morning the crystalline product is filtered off, washed with ethyl acetate and dried. The dry product is thoroughly washed with water, dried and recrystallized from acetonitrile. Thus 1.5 g of 3-[ethyl]-5-[methyl]-2-[2'-(aminoethoxy)-methyl]-4-[2''-(chloro)-phenyl]-6-[methyl]-1,4-di-[hydro]-pyridine-3,5-di-carboxylate] benzenesulfonic acid salt {amlodipine besylate} are obtained, yield 52.9%, mp.: 202 to 203°C.

### Method b)

A mixture of 8.8 g (0.013 mole) of 3-[ethyl]-5-[methyl]--2-[2'-(yl)-ethoxy-methyl]-4-[2''-(chloro)-phenyl]-6-[methyl]-1,4-di-[hydro]-pyridine-3,5-di-[carboxylate]-hexaminium iodide (XI), 10.54 g (0,066 mole) of benzenesulfonic acid (XII), 535 ml of methanol and 535 ml of water is refluxed for an hour. The reaction mixture is allowed to cool to room temperature, poured onto water, extracted with dichloro methane, washed with water, dried and evaporated. The residue is crystallized by storing in acetonitrile in a refrigerator overnight. Thus 4.18 g of 3-[ethyl]-5-[methyl]-2-[2'-(aminoethoxy)-methyl]-4-[2''-(chloro)-phenyl]-6-[methyl]-1,4-di-[hydro]-pyridine-3,5-di-[carboxylate] benzenesulfonic acid salt {amlodipine besylate} are obtained, yield 55.4%, mp.: 205 to 206°C (recrystallized from acetonitrile).

| Elementary analysis: for the Formula C₂₆H₃₁ClN₂O₈S (567.055) : | | | | | |
|---|---|---|---|---|---|
| calculated | C 55.07%, | H 5.51%, | N 4.94%, | Cl 6.25%, | S 5.65%; |
| found | C 54.71%, | H 5.53%, | N 4.95%, | Cl 6,05%, | S 5.57%. |

## Claims

1. Process for preparing 3-[ethyl]-5-[methyl]-2-[2'-(aminoethoxy)-methyl]-4-[2''-(chloro)-phenyl]-6-[methyl]-1,4-di-[hydro]-pyridine-3,5-di-[carboxylate] benzenesulfonic acid salt of Formula 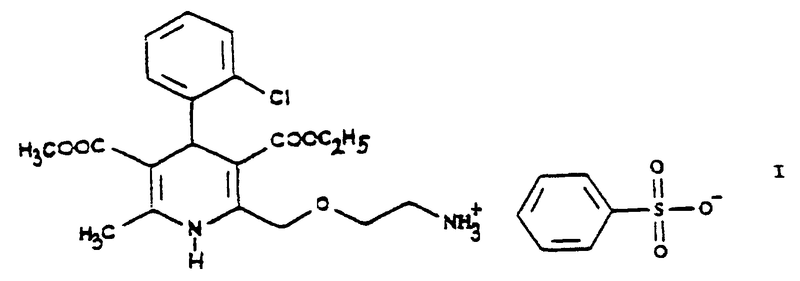 including its optically active isomers, **characterized by**
a₁) reacting 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula 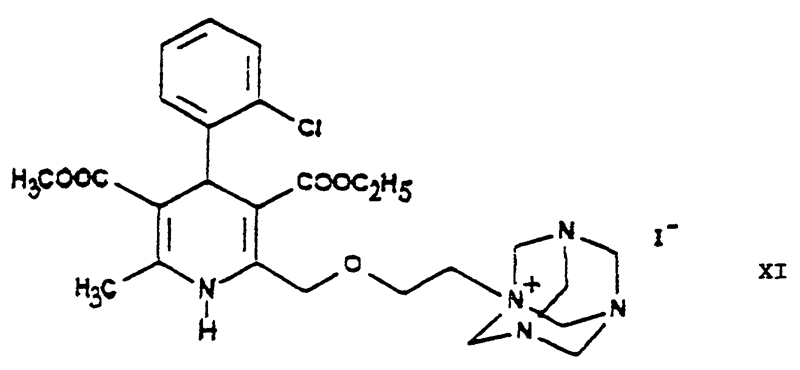 with benzenesulfonic acid of Formula 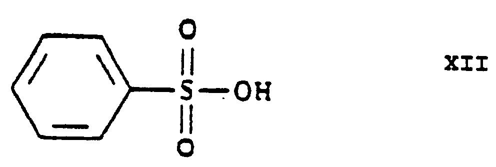 in a mixture of water an 1 or more organic solvent(s);
or
a₂) reacting 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula 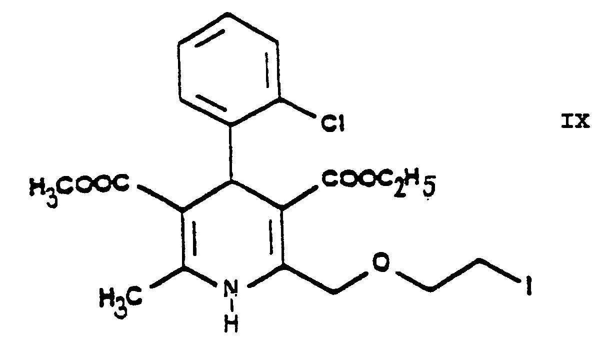 with hexamethylene tetramine of Formula 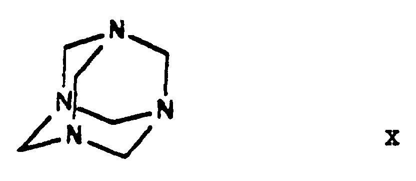 and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
or
a₃) exchanging in 3-[ethyl]-5-[methyl]-2-[2'-(chloro) - ethoxy-methyl]-4-[2''-(chloro)-phenyl]-6-[methyl]-1,4-di-[hydro]-pyridine-3,5-di-[carboxylate] of Formula 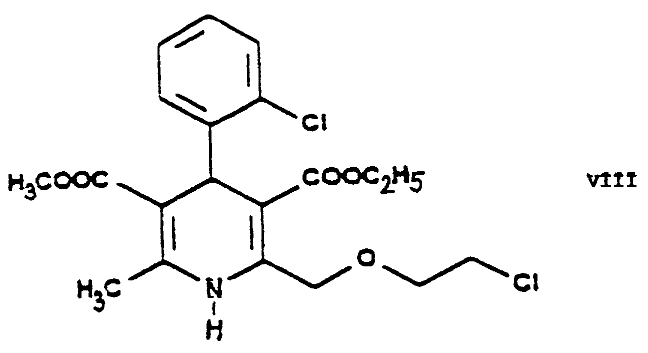 the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
or
a₄) reacting ethyl-4-[2'-(chloro)-ethoxy]-2-[2''-(chloro)-benzylidene]-acetoacetate of Formula 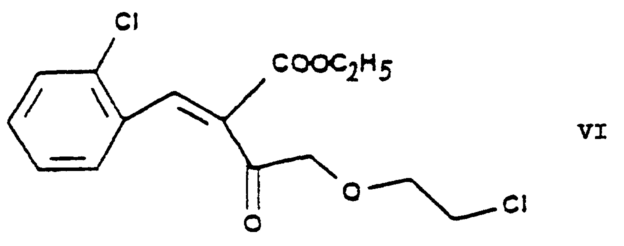 with methyl-3-(amino)-crotonate of Formula 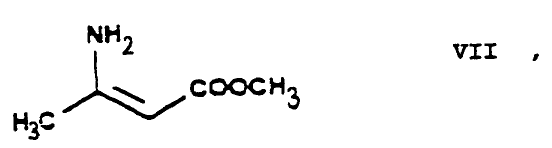 exchanging in the 3-[ethyl]-5-[methyl]-2-[2' - (chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
or
a₅) reacting ethyl-4-[2'-(chloro)-ethoxy]-acetoacetate of Formula 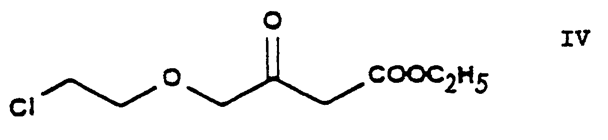 with 2-(chloro)-benzaldehyde of Formula 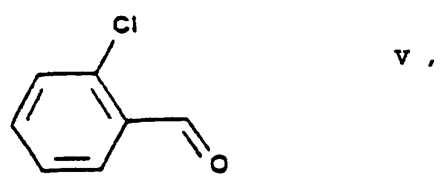 reacting the ethyl-4-[2'-(chloro)-ethoxy]-2-[2''-(chloro)-benzylidene]-acetoacetate of Formula VI thus obtained with methyl-3-(amino)-crotonate of Formula VII, exchanging in the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro) -phenyl] -6-[methyl]-1,4-di-[hydro]-pyridine-3,5-di-[carboxylate]-hexaminium iodide of Formula XI thus obtained with benzenesulfonic acid of Formula XII;
or
a₆) reacting ethyl-4-(bromo)-acetoacetate of Formula 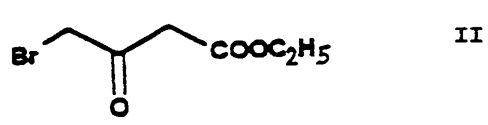 with ethylene chlorohydrine of Formula 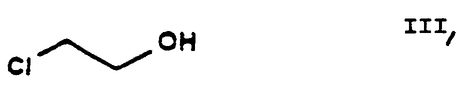 reacting the ethyl-4-[2'-(chloro)-ethoxy]-acetoacetate of Formula IV thus obtained with 2-(chloro)-benzaldehyde of Formula V, reacting the ethyl-4-[2'-(chloro)-ethoxy]-2-[2''-(chloro)-benzylidene]-acetoacetate of Formula VI thus obtained with methyl-3-(amino)-crotonate of Formula VII, exchanging in the 3-[ethyl]-5-[methyl]-2-[2'-chloro)-ethoxy-methyl]-4-[2''-(chloro)-phenyl]-6-[methyl]-1,4-di-[hydro]-pyridine-3,5-di-[carboxylate] of Formula VIII thus obtained the chlorine for iodine, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained with benzenesulfonic acid of Formula XII;
or
a,) exchanging in the ethyl-4-[2'-(chloro)-ethoxy]- acetoacetate of Formula IV the chlorine for iodine, reacting the ethyl-4-[2'-(iodo)-ethoxy]-acetoacetate of Formula 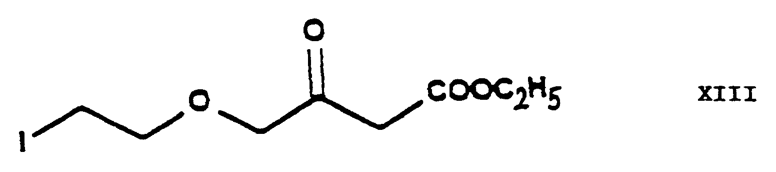 thus obtained with 2-(chloro)-benzaldehyde of Formula V, reacting the ethyl-4-[2'-(iodo)-ethoxy]-2-[2''-(chloro)-benzylidene]-acetoacetate of Formula 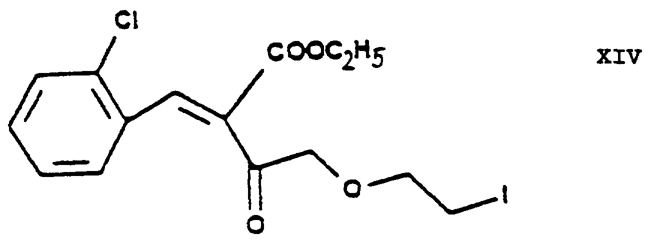 thus obtained with methyl-3-(amino)-crotonate of Formula VII, reacting the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX thus obtained with hexamethylene tetramine of Formula X and reacting the 3-[ethyl]-5-[methyl]-2-[2'-(yl)-ethoxy-methyl]-4-[2''-(chloro)-phenyl]-5-[methyl]-1,4-i-[hydro]-pyridine-3,5-di-[carboxylate]-hexaminium iodide of Formula XI thus obtained with benzenesulfonic acid of Formula XII;
in variants as) and a₆) alternatively reacting the ethyl-4-[2'-(chloro)-ethoxy]-acetoacetate of Formula IV with the 2-(chloro)-benzaldehyde of Formula V and the methyl-3-(amino)-crotonate of Formula VII to the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII without isolating the ethyl-4-[2'-(chloro)-ethoxy]-2-[2''-(chloro)-benzylidene]-acetoacetate of Formula VI,
optionally separating optically active isomers in a manner known per se from the 3-[ethyl]-5-[methyl]-2-[2'-(aminomethoxy)-methyl]-4-[2''-(chloro)-phenyl]-6-[methyl]-1,4-di-[hydro]-pyridine-3,5-di-[carboxylate] benzenesulfonic acid salt of Formula I, the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII, the 3-[ethyl]-5-[methyl]-2-[2'-(iodo)-ethoxy-methyl]-4-[2''-(chloro)-phenyl]-6-[methyl]-1,4-di-[hydro]-pyridine-3,5-di-[carboxylate] of Formula IX or the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI thus obtained and further reacting the optically active isomers of the last three ones thus obtained.

2. Process according to claim 1, **characterized by** using 1 or more water miscible organic solvent(s) as [an] organic solvent(s) for the hydrolysis of the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of the formula XI with benzenesulfonic acid of formula XII.

3. Process according to claim 1 or 2, **characterized by** using 1 or more C₁₋₃-alkanol(s) as [a] water miscible solvent(s) for the hydrolysis of the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of the Formula XI with benzenesulfonic acid of Formula XII.

4. Process according to claims 1 to 3, **characterized by** using 1 or more organic solvent(s) partially miscible or immiscible with water as [an] organic solvent(s) for the hydrolysis of the 3-[ethyl]-5-[methyl]-2-[2'-(yl) - ethoxy-methyl]-4-[2''-(chloro)-phenyl]-6-[methyl]-1,4-di-[hydro]-pyridine-3,5-di-[carboxylate]-hexaminium iodide of Formula XI with benzenesulfonic acid of Formula XII.

5. Process according to claim 1 or 4, **characterized by** using 1 or more C₄₋₈-alkanol(s) particularly n-butanol, and/or ethyl acetate as [an] organic solvent(s) for the hydrolysis of the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of the Formula XI with benzenesulfonic acid of Formula XII.

6. Process according to any of claims 1 to 5, **characterized by** using benzenesulfonic acid of Formula XII in at least a 4 molar amount, related to 1 mole of the 3-[ethyl]-5-[methyl]-2-[2'-(yl)-ethoxy-methyl]-4-[2''-(chloro)-phenyl]-6-[methyl]-1,4-di-[hydro]-pyridine-3,5-di-[carboxylate]-hexaminium iodide of Formula XI.

7. Process according to claim 1, **characterized by** carrying out the reaction of the 3-[ethyl]-5-[methyl]-2-[2'-(iodo)-ethoxy-methyl]-4-[2''-(chloro)-phenyl]-6-[methyl]-1,4-di- [hydro]-pyridine-3,5-di-[carboxylate] of Formula IX with the hexamethylene tetramine of Formula X in 1 or more C₁₋₅-alkanol(s) and/or acetonitrile.

8. Process according to claim 1, **characterized by** carrying out exchanging in the 3-[ethyl]-5-[methyl]-2-[2'-(chloro)-ethoxy-methyl]-4-[2''-(chloro)-phenyl]-6-[methyl]-1,4-di-[hydro]-pyridine-3,5-di-[carboxylate] of Formula VIII chlorine for iodine by reacting it with 1 or more alkali iodide(s), particularly sodium iodide.

9. Process according to claim 1 or 8, **characterized by** carrying out exchanging in the 3-[ethyl]-5-[methyl]-2-[2'-(chloro)-ethoxy-methyl] -4-[2''-(chloro)-phenyl]-6-[methyl]-1,4-di-[hydro]-pyridine-3,5-di-[carboxylate] of Formula VIII chlorine for iodine in 1 or more alkanol(s) having a high boiling point, particularly isopropanol.

10. Process according to claim 1, **characterized by** carrying out the reaction of the ethyl-4-[2'-(chloro)-ethoxy]-2-[2''-(chloro)-benzylidene]-acetoacetate of Formula VI with the methyl-3-(amino)-crotonate of Formula VII in 1 or more C₁₋₄-alkanol(s), particularly methanol, ethanol and/or isopropanol, and/or polar aprotic organic solvent(s), particularly acetonitrile.

11. Process according to claim 1, **characterized by** carrying out the reaction of the ethyl-4-[2'-(chloro)-ethoxy]--acetoacetate of Formula IV with the 2-(chloro)--benzaldehyde of Formula V in the presence of piperidine acetate as catalyst.

12. Process according to claim 1, **characterized by** carrying out exchanging in the ethyl-4-[2'-(chloro)-ethoxy]--acetoacetate of Formula IV the chlorine for iodine by reacting it with 1 or more alkali iodide(s), particularly sodium iodide.

13. Compounds of the Formulae VIII, IX and XI.

## Patentansprüche

1. Verfahren zur Herstellung von 3-[Ethyl]-5-[methyl]-2-[2'-(aminoethoxy)-methyl]-4-[2"-(chlor)-phenyl]-6-[methyl]-1,4-di-[hydro]-pyridin-3,5-di-[carboxylat]benzolsulfonsäuresalz der Formel 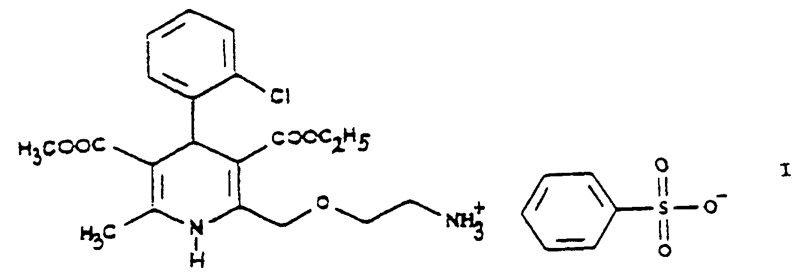 einschließlich seiner optisch aktiven Isomeren, **gekennzeichnet durch**
a₁) Umsetzen von 3-[Ethyl]-5-[methyl]-2-[2'-(yl)-ethoxy-methyl]-4-[2"-(chlor)-phenyl]-6-[methyl]-1,4-di-[hydro]-pyridin-3,5-di[carboxylat]-hexaminiumiodid der Formel 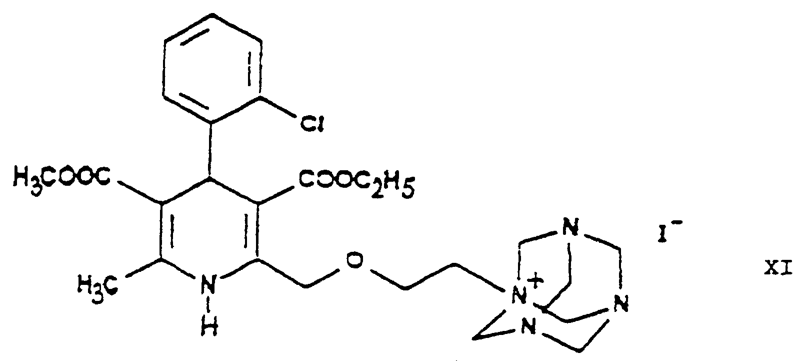 mit Benzolsulfonsäure der Formel 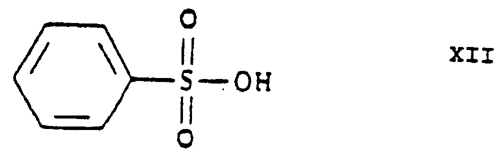 in einer Mischung von Wasser und einem oder mehreren organischen Lösungsmittel(n);
oder
a₂) Umsetzen von 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylat] der Formel 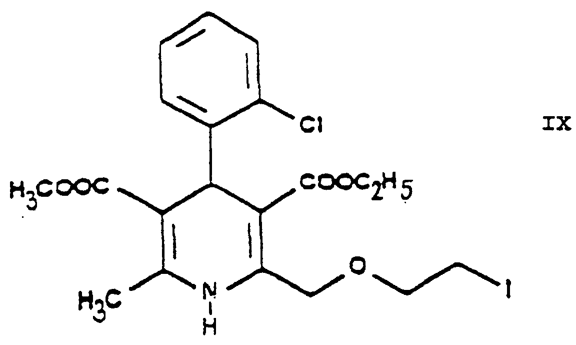 mit Hexamethylentetramin der Formel 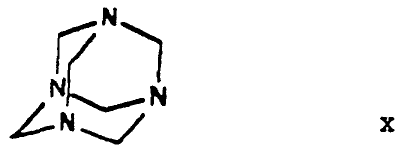 und Umsetzen des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der so erhaltenen Formel XI mit Benzolsulfonsäure der Formel XII;
oder
a₃) Austauschen in 3-[Ethyl]-5-[methyl]-2-[2'-(chlor)-ethoxy-methyl]-4-[2"-(chlor)-phenyl]-6-[methyl]-1,4-di-[hydro]-pyridin-3,5-di-[carboxylat] der Formel 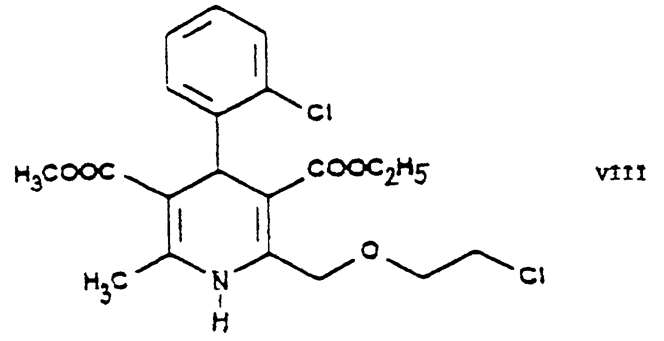 des Chlors **durch** Iod, Umsetzen des so erhaltenen 3-[Ethyl]-5-[methyl]-2-[2'-(iod)-ethoxy-methyl]-4-[2"-(chlor)-phenyl]-6-[methyl]-1,4-di-[hydro]-pyridin-3,5-di[carboxylats] der Formel IX mit Hexamethylentetramin der Formel X und Umsetzen des so erhaltenen 3-[Ethyl]-5-[methyl]-2-[2'-(yl)-ethoxy-methyl]-4-[2"-(chlor)-phenyl-6-[methyl]-1,4-di-[hydro]-pyridin-3,5-di-[carboxylat]-hexaminiumiodids der Formel XI mit Benzolsulfonsäure der Formel XII;
oder
a₄) Umsetzen von Ethyl-4-[2'-(chlor)-ethoxy]-2-[2"-(chlor)-benzyliden]-acetoacetat der Formel 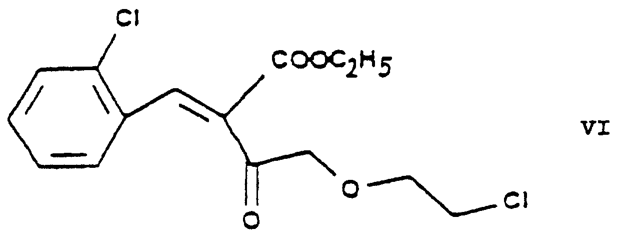 mit Methyl-3-(amino)-crotonat der Formel 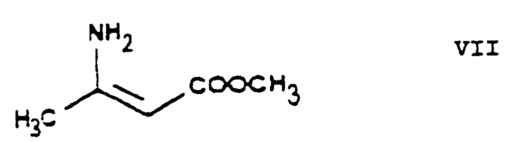 Austauschen in dem so erhaltenen 3-[Ethyl]-5-[methyl]-2-[2'-(chlor)-ethoxy-methyl]-4-[2"-(chlor)-phenyl]-6-[methyl]-1,4-di[hydro]-pyridin-3,5-di-[carboxylat] der Formel VIII des Chlors **durch** Iod, Umsetzen des so erhaltenen 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylats] der Formel IX mit Hexamethylentetramin der Formel X und Umsetzen des so erhaltenen 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII;
oder
a₅) Umsetzen von Ethyl-4-[2'-(chlor)-ethoxy]-acetoacetat der Formel 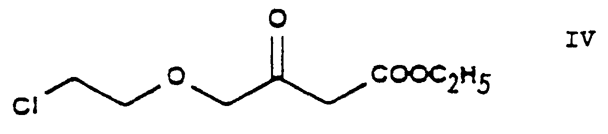 mit 2-(Chlor)-benzaldehyd der Formel 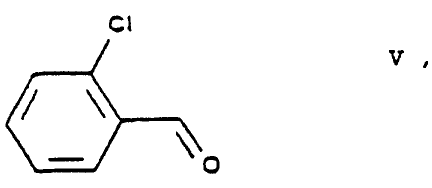 Umsetzen des so erhaltenen Ethyl-4-[2'-(chlor)-ethoxy]-2-[2"-(chlor)-benzyliden]-acetoacetats der Formel VI mit Methyl-3-(amino)-crotonat der Formel VII, Austauschen in dem so erhaltenen 3-[Ethyl]-5-[methyl]-2-[2'-(chlor)-ethoxy-methyl]-4-[2"-(chlor)-phenyl]-6-[methyl]-1,4-di-[hydro]-pyridin-3,5-di[carboxylat] der Formel VIII des Chlors **durch** Iod, Umsetzen des so erhaltenen 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylats] der Formel IX mit Hexamethylentetramin der Formel X und Umsetzen des so erhaltenen 3-[Ethyl]-5-[methyl]-2-[2'-(yl)-ethoxymethyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII;
oder
a₆) Umsetzen von Ethyl-4-(brom)-acetoacetat der Formel 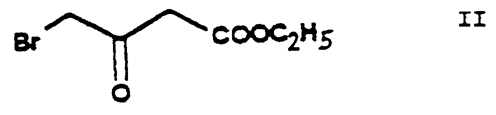 mit Ethylenchlorhydrin der Formel 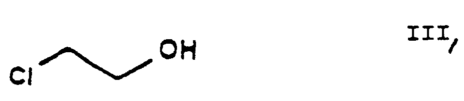 Umsetzen des so erhaltenen Ethyl-4-[2'-(chlor)-ethoxy]-acetoacetats der Formel IV mit 2-(Chlor)-benzaldehyd der Formel V, Umsetzen des so erhaltenen Ethyl-4-[2'-(chlor)-ethoxy]-2-[2"-(chlor)-benzyliden]-acetoacetats der Formel VI mit Methyl-3-(amino)-crotonat der Formel VII, Austauschen in dem so erhaltenen 3-[Ethyl]-5-[methyl]-2-[2'-chlor)-ethoxy-methyl]-4-[2"-(chlor)-phenyl]-6-[methyl]-1,4-di-[hydro]-pyridin-3,5-di-[carboxylat] der Formel VIII des Chlors **durch** Iod, Umsetzen des so erhaltenen 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylats] der Formel IX mit Hexamethylentetramin der Formel X und Umsetzen des so erhaltenen 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII;
oder
a₇) Austauschen in dem Ethyl-4-[2'-(chlor)-ethoxy]-acetoacetat der Formel IV des Chlors **durch** Iod, Umsetzen des so erhaltenen Ethyl-4-[2'-(iod)-ethoxy]-acetoacetats der Formel 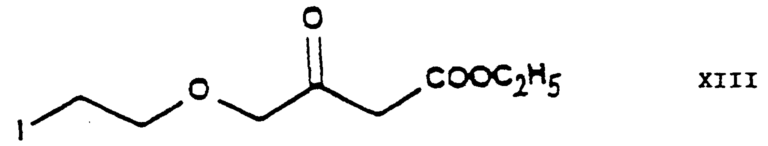 mit 2-(Chlor)-benzaldehyd der Formel V, Umsetzen des so erhaltenen Ethyl-4-[2'-(iod)-ethoxy]-2-[2"-(chlor)-benzyliden]-acetoacetats der Formel 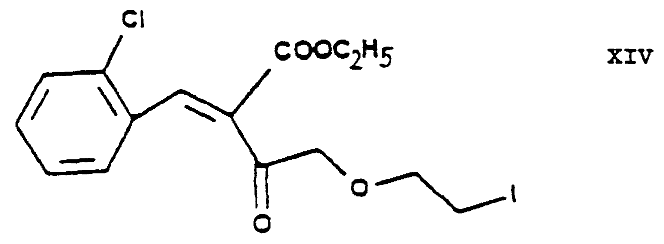 mit Methyl-3-(amino)-crotonat der Formel VII, Umsetzen des so erhaltenen 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylats] der Formel IX mit Hexamethylentetramin der Formel X und Umsetzen des so erhaltenen 3-[Ethyl]-5-[methyl]-2-[2'-(yl)-ethoxy-methyl]-4-[2"-(chlor)-phenyl]-6-[methyl]-1,4-i-[hydro]-pyridin-3,5-di-[carboxylat]-hexaminiumiodids der Formel XI mit Benzolsulfonsäure der Formel XII;
wobei in den Varianten a₅) und a₆) alternativ das Ethyl-4-[2'-(chlor)-ethoxy]-acetoacetat der Formel IV mit dem 2-(Chlor)-benzaldehyd der Formel V und das Methyl-3-(amino)-crotonat der Formel VII zu dem 3-[Ethyl]-5-[methyl]-2-[2'-(chlor)-ethoxy-methyl]-4-[2"-(chlor)-phenyl]-6-[methyl]-1,4-di-[hydro]-pyridin-3,5-di-[carboxylat] der Formel VIII ohne Isolieren des Ethyl-4-[2'-(chlor)-ethoxy]-2-[2"-(chlor)-benzyliden]-acetoacetats der Formel VI umgesetzt wird,
wahlweises Abtrennen von optisch aktiven Isomeren in einer per se bekannten Weise von dem so erhaltenen 3-[Ethyl]-5-[methyl]-2-[2'-(aminomethoxy)-methyl]-4-[2"-(chlor)-phenyl]-6-[methyl]-1,4-di-[hydro]-pyridin-3,5-di-[carboxylat]benzolsulfonsäuresalz der Formel I, dem 3-[Ethyl]-5-[methyl]-2-[2'-(chlor)-ethoxy-methyl]-4-[2"-(chlor)-phenyl]-6-[methyl]-1,4-di-[hydro]-pyridin-3,5-di-[carboxylat] der Formel VIII, dem 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylat] der Formel IX oder dem 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodid der Formel XI, und weiter Umsetzen der optisch aktiven Isomere von den so erhaltenen letzten Dreien.

2. Verfahren gemäß Anspruch 1, **gekennzeichnet durch** die Verwendung von einem oder mehreren mit Wasser vermischbaren organischen Lösungsmitteln als (ein) organische(s) Lösungsmittel für die Hydrolyse des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII.

3. Verfahren gemäß Anspruch 1 oder 2, **gekennzeichnet durch** die Verwendung von einem oder mehreren C₁₋₃-Alkanol(en) als (einem) mit Wasser vermischbaren Lösungsmittel(n) für die Hydrolyse des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodid der Formel XI mit Benzolsulfonsäure der Formel XII.

4. Verfahren gemäß den Ansprüchen 1 bis 3, **gekennzeichnet durch** die Verwendung von einem oder mehreren organischen Lösungsmitteln, welche mit Wasser teilweise mischbar oder unmischbar sind, als (ein) organische(s) Lösungsmittel für die Hydrolyse des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII.

5. Verfahren gemäß Anspruch 1 oder 4, **gekennzeichnet durch** die Verwendung von einem oder mehreren C₄₋₈-Alkanol(en), insbesondere n-Butanol und/oder Ethylacetat als (einem) organischen Lösungsmittel für die Hydrolyse des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI mit Benzolsulfonsäure der Formel XII.

6. Verfahren gemäß mindestens einem der Ansprüche 1 bis 5, **gekennzeichnet durch** die Verwendung von Benzolsulfonsäure der Formel XII in mindestens 4-molarer Menge in bezug auf 1 Mol des 3-[Ethyl]-5-[methyl]-2-[2'-(yl)-ethoxy-methyl]-4-[2"-(chlor)-phenyl]-6-[methyl]-1,4-di-[hydro]-pyridin-3,5-di-[carboxylat]-hexaminiumiodids der Formel XI.

7. Verfahren gemäß Anspruch 1, **gekennzeichnet durch** die Durchführung der Reaktion des 3-[Ethyl]-5-[methyl]-2-[2'-(iod)-ethoxy-methyl]-4-[2"-(chlor)-phenyl]-6-[methyl]-1,4-di-[hydro]-pyridin-3,5-di-[carboxylats] der Formel IX mit dem Hexamethylentetramin der Formel X in einem oder mehreren C₁₋₅-Alkanol(en) und/oder Acetonitril.

8. Verfahren gemäß Anspruch 1, **gekennzeichnet durch** die Durchführung des Austausches in dem 3-[Ethyl]-5-[methyl]-2-[2'-(chlor)-ethoxy-methyl]-4-[2"-(chlor)-phenyl]-6-[methyl]-1,4-di[hydro]-pyridin-3,5-di-[carboxylat] der Formel VIII von Chlor **durch** Iod, indem es mit einem oder mehreren Alkaliiodid(en), insbesondere Natriumiodid, umgesetzt wird.

9. Verfahren gemäß Anspruch 1 oder 8, **gekennzeichnet durch** die Durchführung des Austausches in dem 3-[Ethyl]-5-[methyl]-2-[2'-(chlor)-ethoxy-methyl]-4-[2"-(chlor)-phenyl]-6-[methyl]-1,4-di-[hydro]-pyridin-3,5-di-[carboxylat] der Formel VIII von Chlor **durch** Iod in einem oder mehreren Alkanol(en) mit einem hohen Siedepunkt, insbesondere Isopropanol.

10. Verfahren gemäß Anspruch 1, **gekennzeichnet durch** die Durchführung der Reaktion des Ethyl-4-[2'-(chlor)-ethoxy]-2-[2"-(chlor)-benzyliden]-acetoacetats der Formel VI mit dem Methyl-3-(amino)-crotonat der Formel VII in einem oder mehreren C₁₋₄-Alkanol(en), insbesondere Methanol, Ethanol und/oder Isopropanol, und/oder (einem) polar aprotischen organischen Lösungsmittel(n), insbesondere Acetonitril.

11. Verfahren gemäß Anspruch 1, **gekennzeichnet durch** die Durchführung der Reaktion des Ethyl-4-[2'-(chlor)-ethoxy]-acetoacetats der Formel IV mit dem 2-(Chlor)-benzaldehyd der Formel V in Gegenwart von Piperidinacetat als Katalysator.

12. Verfahren gemäß Anspruch 1, **gekennzeichnet durch** die Durchführung des Austausches in dem Ethyl-4-[2'-(chlor)-ethoxy]-acetoacetat der Formel IV von dem Chlor **durch** Iod **durch** die Umsetzung mit einem oder mehreren Alkaliiodid(en), insbesondere Natriumiodid.

13. Verbindungen der Formel VIII, IX und XI.

## Revendications

1. Procédé pour la préparation du sel d'acide benzène sulfonique du 3-[éthyl]-5-[méthyl]-2-[2'-(aminoéthoxy)-méthyl]-4-[2"-(chloro)-phényl]-6-[méthyl]-1,4-di-[hydro]-pyridine-3,5-di-[carboxylate] de Formule (I) : 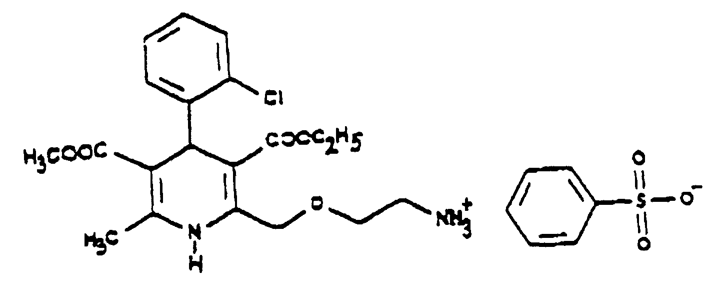 y compris de ses isomères optiquement actifs, **caractérisé par** :
(a₁) la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de Formule (XI) : 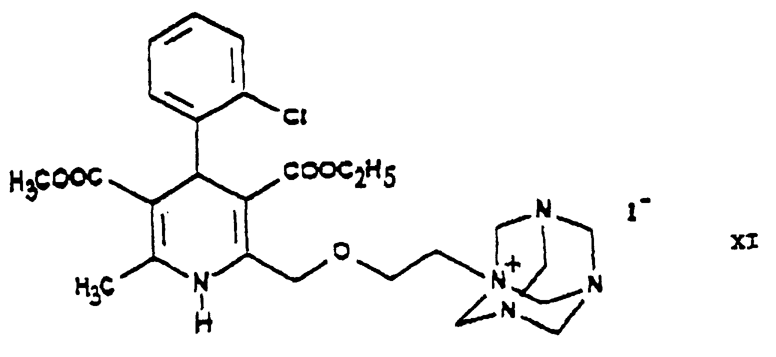 avec l'acide benzène sulfonique de Formule (XII) : 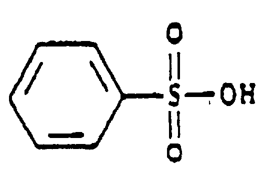 dans un mélange d'eau et d'un ou plusieurs solvants organiques ; ou
(a₂) la réaction du 3-[éthyl]-5-[méthyl]-2-[2'-(iodo)-éthoxy-méthyl]-4-[2"-(chloro)-phényl]-6-[méthyl]-1,4-di-[hydre]-pyridine-3,5-di-[carboxylate] de Formule (IX) 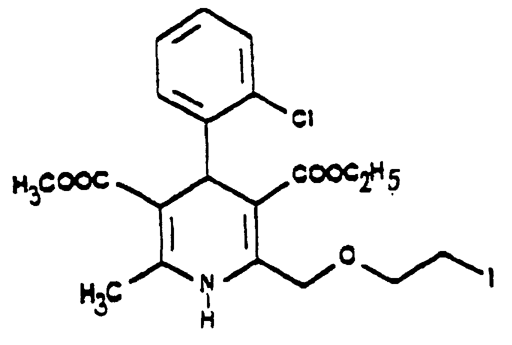 avec de l'hexaméthylène tétramine de Formule (X) : 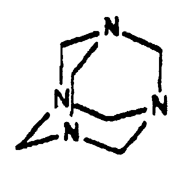 et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de Formule (XI) ainsi obtenu avec de l'acide benzène sulfonique de Formule (XII) ; ou
(a₃) l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) : 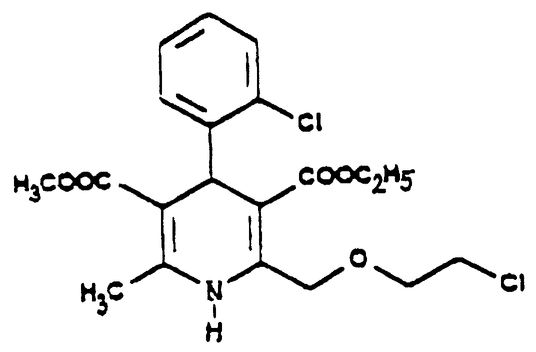 de l'atome de chlore par un atome d'iode, la réaction du 3-[éthyl]-5-[méthyl]-2-[2'-(iodo)-éthoxy-méthyl]-4-[2"-(chloro)-phényl]-6-[méthyl]-1,4-di-[hydro]-pyridine-3,5-di-[carboxylate] de Formule (IX) ainsi obtenu avec de l'hexaméthylène tétramine de Formule (X) et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) ainsi obtenu avec de l'acide benzène sulfonique de Formule (XII) ; ou
(a₄) la réaction du 4-[2'-(chloro)-éthoxy]-2-[2"-(chloro)-benzylldène]- acétoacétate d'éthyle de Formule (VI) : 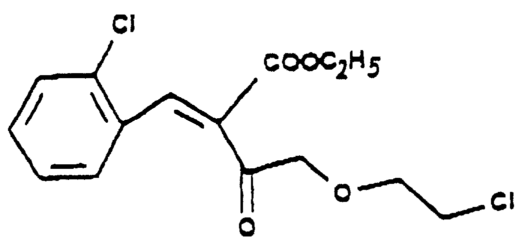 avec du 3-(amino)-crotonate de méthyle de Formule (VII) : 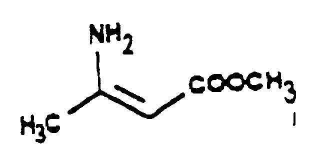 l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) ainsi obtenu, de l'atome de chlore par un atome d'iode, la réaction du 3-[éthyl]-5-[méthyl]-2-[2'-(iodo)-éthoxy-méthyl]-4-[2"-(chloro)-phényl]-6-[méthyl]-1,4-di-[hydro]-pyridine-3,5-di-[carboxylate] de Formule (IX) ainsi obtenu avec de l'hexaméthylène tétramine de Formule (X) et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) ainsi obtenu avec de l'acide benzène sulfonique de Formule (XII) ; ou
(a₅) la réaction du 4-[2'-(chlore)-éthoxy]-acétoacétate d'éthyle de Formule (IV) : 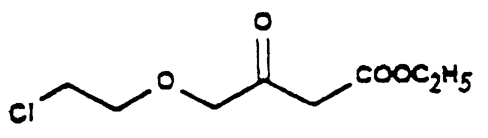 avec du 2-(chloro)-benzaldéhyde de Formule (V) : 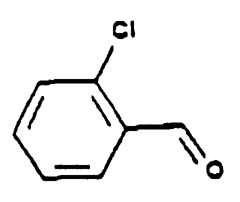 la réaction du 4-[2'-(chloro)-éthoxy]-2-[2"-(chloro)-benzylidène]- acétoacétate d'éthyle de Formule (VI) ainsi obtenu avec du 3-(amino)-crotonate de méthyle de Formule (VII), l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) ainsi obtenu, de l'atome de chlore par un atome d'iode, la réaction du 3-[éthyl]-5-[méthyl]-2-[2'-(iodo)-éthoxy-méthyl]-4-[2"-(chloro)-phényl]-6-[méthyl]-1,4-di-[hydro]-pyridine-3,5-di-[carboxylate] de Formule (IX) ainsi obtenu, avec de l'hexaméthylène tétramine de Formule (X) et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) ainsi obtenu, avec de l'acide benzène sulfonique de Formule (XII) ; ou
(a₆) la réaction du 4-(bromo)-acétoacétate d'éthyle de Formule (II) : 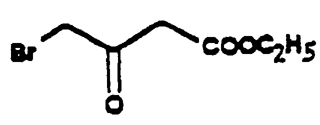 avec de l'éthylène chlorohydrine de Formule (III) : 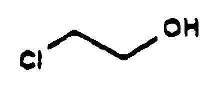 la réaction du 4-[2'-(chloro)-éthoxy]-acétoacétate d'éthyle de Formule (IV) ainsi obtenu, avec du 2-chlorobenzaldéhyde de Formule (V), la réaction 4-[2'-(chloro)-éthoxy]-2-[2"-(chloro)-benzylidène]-acétoacétate d'éthyle de Formule (VI) ainsi obtenu avec du 3-(amino)-crotonate de méthyle de Formule (VII), l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) ainsi obtenu, de l'atome de chlore par un atome d'lode, la réaction du 3-[éthyl]-5-[méthyl]-2-[2'-(iodo)-éthoxy-méthyl]-4-[2"-(chloro)-phényl]-6-[méthyl]-1,4-di-[hydro]-pyridine-3,5-di-[carboxylate] de Formule (IX) ainsi obtenu, avec de l'hexaméthylène tétramine de Formule (X) et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) ainsi obtenu avec de l'acide benzène sulfonique de Formule (XII) ; ou
(a₇) l'échange dans le 4-[2'-(chloro)-éthoxy]-acétoacétate d'éthyle de Formule (IV), de l'atome de chlore par un atome d'iode, la réaction du 4-[2'-(iodo)-éthoxy]-acétoacétate d'éthyle de Formule (XIII) 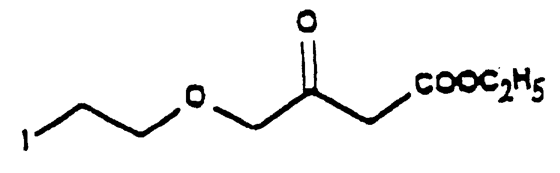 ainsi obtenu avec le 2-(chloro)-benzaldéhyde de Formule (V), la réaction du 4-[2'-(iodo)-éthoxy]-2-[2"-(chloro)-benzylidène]-acétoacétate d'éthyle de Formule (XIV) 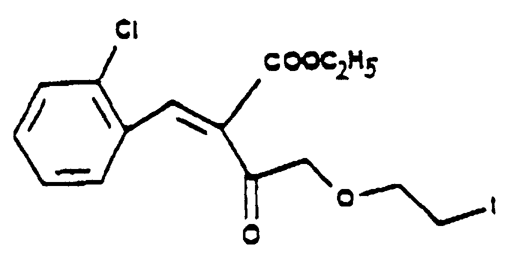 ainsi obtenu avec du 3-(amino)-crotonate de méthyle de Formule (VII), la réaction du 3-[éthyl]-5-[méthyl]-2-[2'-(iodo)-éthoxy-méthyl]-4-[2"-(chloro)-phényl]-6-[méthyl]-1,4-di-[hydro]-pyridine-3,5-di-[carboxylate] de Formule (IX) ainsi obtenu, avec de l'hexaméthylène tétramine de Formule (X) et la réaction de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]. hexaminium de formule (XI) ainsi obtenu, avec de l'acide benzène sulfonique de Formule (XII) ;
dans les variantes (a₅) et (a₆), dans une variante, la réaction du 4-[2'-(chloro)-éthoxy]-acétoacétate d'éthyle de Formule IV avec le 2-(chloro)-benzaldényde de Formule (V) et le 3-(amino)-crotonate de méthyle de Formule (VII) pour donner le 3-[éthyl]-5-[méthyl]-2-[2'-(chloro)-éthoxy-méthyl]-4-[2"-(chloro)-phényl]-6-[méthyl]-1,4-di-[hydro]-pyrldine-3,5-di-[carboxylate] de Formule (VIII) sans isoler le 4-[2'-(chlore)-éthoxy]-2-[2"-(chloro)-benzylidène]-acétoacétate d'éthyle de Formule (VI),
éventuellement, la séparation des isomères optiquement actifs d'une manière connue en soi à partir du sel d'acide benzène sulfonique de 3-[éthyl]-5-[méthyl]-2-[2'-(aminoéthoxy)-méthyl]-4-[2"-(chloro)-phényl]-6-[méthyl]-1,4-di-[hydro]-pyridine-3,5-di-[carboxylate] de Formule (I), du 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII), du 3-[éthyl]-5-[méthyl]-2-[2'-(iode)-éthoxy-méthyl]-4-[2"-(chloro)-phényl]-6-[méthyl]-1,4-di-[hydro]-pyridine-3,5-di-[carboxylate] de Formule (IX) ou de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) ainsi obtenu et ensuite, la réaction des isomères optiquement actifs des trois derniers composés ainsi obtenus.

2. Procédé suivant la revendication 1, **caractérisé par** l'utilisation d'un ou de plusieurs solvants organiques miscibles à l'eau en tant que solvant ou solvants organiques pour l'hydrolyse de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) avec de l'acide benzène sulfonique de Formule (XII).

3. Procédé suivant les revendications 1 ou 2, **caractérisé par** l'utilisation d'un ou de plusieurs alcanols en C₁₋ ₃ en tant que solvant ou solvants miscibles à l'eau pour l'hydrolyse de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) avec de l'acide benzène sulfonique de Formule (XII).

4. Procédé suivant les revendications 1 à 3, **caractérisé par** l'utilisation d'un ou de plusieurs solvants organiques partiellement miscibles ou immiscibles à l'eau en tant que solvant ou solvants organiques pour l'hydrolyse de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) avec de l'acide benzène sulfonique de Formule (XII).

5. Procédé suivant les revendications 1 ou 4, **caractérisé par** l'utilisation d'un ou de plusleurs alcanols en C₄₋ ₈ en particulier le n-butanol, et/ou d'acétate d'éthyle en tant que solvant ou solvants organiques pour l'hydrolyse de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI) avec de l'acide benzène sulfonique de Formule (XII).

6. Procédé suivant l'une quelconque des revendications 1 à 5, **caractérisé par** l'utilisation de l'acide benzène sulfonique de Formule (XII) en une quantité au moins 4 fois molaire, par rapport à 1 mole de l'iodure de 3-[éthyl]-5-[méthyl]-2-[2'-(yl)-éthoxy-méthyl]-4-[2"-(chloro)-phényl]-6-[méthyl]-1,4-di-[hydro]-pyridine-3,5-di-[carboxylate]-hexaminium de formule (XI).

7. Procédé suivant la revendication 1, **caractérisé par** la réalisation de la réaction du 3-[éthyl]-5-[méthyl]-2-[2'-(iodo)-éthoxy-méthyl]-4-[2"-[chloro)-phényl]-6-[méthyl]-1,4-di-[hydro]-pyridine-3,5-di-[carboxylate] de Formule (IX) avec de l'hexaméthylène tétramine de Formule (X) dans un ou plusieurs alcanols en C₁₋₆ et/ou de l'acétonitrile.

8. Procédé suivant la revendication 1, **caractérisé par** la réalisation de l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) de l'atome de chlore par un atome d'iode par réaction de celui-cl avec un ou plusleurs iodures d'alcalin, en particulier l'iodure de sodium.

9. Procédé suivant les revendications 1 ou 8, **caractérisé par** la réalisation de l'échange dans le 3-[éthyl]-5-[méthyl]-2-[2'-(chloro)-éthoxy-méthyl]-4-[2"-(chloro)-phényl]-6-[méthyl]-1,4-di-[hydro]-pyridine-3,5-di-[carboxylate] de Formule (VIII) de l'atome de chlore par un atome d'lode dans un ou plusieurs alcanols ayant un point d'ébullition élevé, en particulier de l'isopropanol.

10. Procédé suivant la revendication 1, **caractérisé par** la réalisation de la réaction du 4-[2'-(chloro)-éthoxy]-2-[2"-(chloro)-benzylidène]-acétoacétate d'éthyle de Formule (VI) avec du 3-(amino)-crotonate de méthyle de Formule (VII), dans un ou plusieurs alcanols en C₁₋₄, en particulier le méthanol, l'éthanol et/ou l'isopropanol, et/ou un ou des solvants organiques aprotiques polaires, en particulier l'acétonitrile.

11. Procédé suivant la revendication 1, **caractérisé par** la réalisation de la réaction du 4-[2'-(chloro)-éthoxy]-acétoacétate d'éthyle de Formule IV avec le 2-(chloro)-benzaldéhyde de Formule (V) en présence d'acétate de pipéridine en tant que catalyseur.

12. Procédé suivant la revendication 1, **caractérisé par** la réalisation de l'échange dans le 4-[2'-(chloro)-éthoxy]-acétoacétate d'éthyle de Formule (IV), de l'atome de chlore par un atome d'iode par réaction de celui-ci avec un ou plusleurs iodures d'alcalin, en particulier l'iodure de sodium.

13. Composés des Formules VIII, IX et XI.
